# EUROPEAN PATENT SPECIFICATION

(11) **EP 4 346 771 B1**
(45) Date of publication and mention of the grant of the patent: **19.11.2025**
(21) Application number: 22719934.6
(22) Date of filing: 01.04.2022
(51) Int. Cl.: A61K 9/1277, A61K 8/14, A61K 8/87, A61Q 19/00, A61K 9/113, A61K 31/4745, A61K 31/555, A61K 8/06, A61K 38/12, A61K 47/10, A61K 47/24, A61K 47/28, A61K 47/34, C07K 16/30

(54) **NANOTECHNOLOGICAL PLATFORM BASED ON POLYURETHANE/POLYUREA CHEMISTRY TO FURNISH WATER-OIL-WATER MULTI WALLED AND FUNCTIONALIZABLE NANOCAPSULES AND LIPOSOMES AND THEIR PREPARATION PROCESS**
NANOTECHNOLOGISCHE PLATTFORM AUF BASIS VON POLYURETHAN/POLYHARNSTOFF-CHEMIE ZUR ZUFUHR VON WASSER-ÖL-WASSER-MEHRWANDIGEN UND FUNKTIONALISIERBAREN NANOKAPSELN UND LIPOSOMEN UND VERFAHREN ZU IHRER HERSTELLUNG
PLATEFORME NANOTECHNOLOGIQUE BASÉE SUR LA CHIMIE DES POLYURÉTHANES/POLYURÉES POUR FOURNIR DES NANOCAPSULES ET LIPOSOMES EAU-HUILE-EAU À PAROIS MULTIPLES ET FONCTIONNALISABLES ET LEUR PROCÉDÉ DE PRÉPARATION

(30) Priority: 03.06.2021 EP 21382499
(43) Date of publication of application: 10.04.2024
(73) Proprietor: Ecopol Tech, S.L., 43720 L'Arboç (ES)
(72) Inventor: ROCAS SOROLLA, José, 08860 CASTELLDEFELS (ES); BONELLI BLASCO, Joaquin Daniel, 08004 BARCELONA (ES)
(74) Representative: ZBM Patents - Zea, Barlocci & Markvardsen
(86) International application number: PCT/EP2022/058801
(87) International publication number: WO 2022/253483

(56) References cited:
- WO-A1-01/78888
- WO-A2-2014/114838
- US-A1- 2004 115 254

## Description

### Technical Field

The invention relates to water-oil-water multiwalled nanoencapsulates, liposomes, their preparation process, as well as cosmetic or pharmaceutical compositions comprising them.

### Background Art

Single encapsulated emulsions have been widely used in fabrication of cosmetic products, pharmaceutical products (e.g., vesicles for drug delivery), foodstuff and detergents. Generally, they are formulated with an aqueous continuous phase and a dispersed encapsulated oil phase (O/W emulsions) and for this reason are more suitable for oil soluble active materials than water soluble active materials.

Water-in-oil-in-water (W/O/W) emulsions are also known. They consist of internal water droplets dispersed within larger oil droplets, which themselves have been dispersed in an external aqueous continuous phase.

It is known that a multiple emulsion can be obtained by emulsification procedures at high concentrations of the dispersed phase or the emulsifying agent, due to either phase inversion or to the migration of the emulsifying agent between the two phases of the emulsion. They can be prepared by a two-step emulsification method in the presence of surfactants. The first step in emulsification, provides a water-in-oil (W/O) emulsion, which is then mixed with an aqueous solution to form the W/O/W emulsion.

Thus, WO0178888 A1 relates to W/O/W emulsions based on first to form a water-in-oil-emulsion and then to emulsify the water-in-oil-emulsion into a continuous phase, using external emulsifiers. It discloses the formation of the polymer wall by reaction of two or more polymer precursors that can be isocyanate and amine polymer precursors and using polyurethanes and polyurea chemistry among other types of reaction that occur in the interphase.

US4534783A disclose a process for encapsulation, and particularly to the production of small or minute capsules constituted by a skin or thin wall of polymeric material e.g., polyurea, polyamide, polysulfonamide, polyester, polycarbonate, or polyurethane, which involves providing a) an organic liquid (continuous phase liquid) containing an oil soluble alkylated polyvinylpyrrolidone emulsifier, b) a discontinuous (aqueous) phase liquid containing a water-soluble material, which is the material to be encapsulated, plus a first shell wall component; the discontinuous phase being dispersed in the continuous phase liquid to form a water-in-oil emulsion. A second shell wall component is added to the water-in-oil emulsion whereupon the first shell wall component reacts with the second shell wall component to form a solid polymeric shell wall about the material to be encapsulated. The capsules formed may be directly used as in the form of a suspension of microcapsules in the organic liquid.

US 2004/0115254 A1 also discloses compositions and methods for making water-in-oil-in-water (W/O/W) microparticles. The microparticle comprises an active agent such as nucleic acid or DNA encapsulated in an aqueous interior, an amphiphilic binding molecule such as a cationic lipid, and an encapsulation material such as polyurethanes. The amphiphilic molecule is said to have a first functionality having affinity for the active agent and a second functionality which is soluble in the same solvent as the encapsulation material. The process comprises: a) admixing a first aqueous solution having an active agent with an organic solvent having an encapsulation material to form an emulsion; admixing an amphiphilic binding molecule with the emulsion to form an amphiplex; the amphiphilic molecule comprises a first functionality having affinity for the active agent and a second functionality soluble in the same solvent as the encapsulation material; and c) admixing the amphiplex with a second aqueous solution having a stabilizing agent to form a particle.

However, the emulsification process in W/O/W emulsions is strongly affected by the preparation method and a very different droplet size distribution is achieved which is strictly linked to the product stability. Very often these systems are difficult to stabilize. Besides, the use of dispersing and/or emulsifying agents to form the emulsions can hinder the wall formation process.

On the other hand, encapsulation using polyurethane-polyurea prepolymers have been disclosed to carry out W/O encapsulations. These prepolymers are, simultaneously, prepolymers of the polymer that will form part of the microencapsulate in W/O and act as emulsifying agents, avoiding the use of external emulsifiers and improving the barrier effect of the wall.

For instance, EP2992953A2 discloses a method to prepare small size microcapsules using an amphiphilic self-emulsifying polymer with NCO functional groups that reacts at the same time to form a polymeric wall in O/W systems through phase inversion. The NCO groups are suitable for forming urethane and /or urea type bonds when they react with alcohol and amine functional groups respectively.

Cusco et al., in "Multisensitive drug-loaded polyurethane/polyurea nanocapsules with pH-synchronize shell cationization and redox-triggered release", Polymer Chemistry 2016, vol. 7, pp. 6457-6466 also discloses a one-pot procedure to prepare polyurethane/polyurea polymers to nanoencapsulate different hydrophobic drugs in aqueous conditions. The document discloses polyurethane/polyurea polymers with amine terminal groups. These prepolymers are formed from a diisocyanate which reacts with different functional diols and diamines leading to a self-emulsifiable reactive prepolymer. These prepolymers are then used to nano emulsify the desired hydrophobic drug under aqueous conditions. However, these encapsulations without using external emulsifiers have only been used in systems with only one encapsulation such as O/W encapsulation.

From what it is known in the art, it is derived that there is still the need of providing nanostructures W/O/W with improved properties such as structural stability, increased drug loading, enhanced biocompatibility, or specific biological performance.

### Summary of the invention

Inventors have developed multiwalled nanoencapsulates prepared by water-oil-water encapsulation thanks to the use of specific new polyurethane/polyurea prepolymers capped with amine terminal groups terminal groups that allow to obtain a first encapsulate W/O which is very small, and which is adapted to be able to carry out the second encapsulation in specific conditions that allow to obtain very small (20-50 nm) multiwalled W/O/W encapsulates with high crosslinking and stability and where the degree of crosslinking and of stratification can be modulated.

Advantageously, such encapsulations do not require external emulsifiers and allow a controlled stratification at the nano-interface driven by hydrophobic/hydrophilic gradient in the w/o interface or o/w interface in a two-step nanoencapsulation with two robust walls; the first one created in the w/o interface and the second one created in the o/w interface. The functionality is tunable in their multiwalled structure. Advantageously, the present invention provides a nanotechnological platform for encapsulation which is very versatile.

The nanoencapsulates are obtained by a process based on phase inversion emulsions using self-emulsifiable polyurethane/polyurea reactive prepolymers (pre-designed reactive surfactants) with distinctive hydrophobic/hydrophilic balance to create the w/o emulsion in the first step and the o/w emulsion in the second step. These pre-polymers can be ended (capped) by an amine or by and isocyanate group and can be of different functionally (mainly functionality 2 or around 2 to be able to self-stratify in the specific interface to be formed in the first or second step). Formed walls have been crosslinked, to give them robustness separately, by reacting them with desired amino or isocyanate groups. The pre-design of every prepolymer and its capping function for every step will define the stratification and the robustness of every wall. This methodology gives the possibility to work in interfaces (w/o or o/w) with self-emulsification systems with stoichiometric reactions, minimizing impurities and secondary reactions. No purifications steps are needed between walls preparations.

The use of self-emulsifiable prepolymers in O/W systems to prepare polyurethane/polyureas nanocapsules have already been described, however, it is unexpected that these O/W nanoencapsulations work properly when the dispersed phase is not an oil droplet but a W/O nanoencapsulate which have a size quite higher than an oil droplet.

This has been possible thanks to the selection of the specific prepolymers used in the first W/O encapsulation which have a strong influence in the formation of small W/O nano encapsulate allowing its subsequently use to prepare the W/O/W encapsulations without the use of external surfactants. Besides, this first encapsulation is carried out using a solvent immiscible in water, in particular, a non-polar solvent (e.g., cyclohexane) instead of a polar solvent (e.g., THF) and, preferably, with a certain rotation speed in order to form the emulsion.

The W/O/W nanocapsules obtained can be very small, even having a size around 20-50 nm, with high crosslinking and stability and where the degree of crosslinking and of stratification can be modulated.

Thus, the nanocapsules of the present invention have two separately functionalizable robust walls that effectively separate the inside from the outside, that can be released kinetically at different times, and that can provide a hydrophobic capsule after losing the more hydrophilic outermost layer are disclosed. This is advantageous because this hydrophobic capsule may enter for instance the lymphatic system to reach specific organs or to circulate through the lymph itself, thanks to the fact that it can first enter the blood in a stable way because of its hydrophilic external layer that is not detectable by the reticuloendothelial system (RES).

In some of the applications the most inner wall of the W/O/W system can be formed by a polyurethane/polyurea based polymer, but the outer wall resembles the structure of a liposome, thus, having in common the first encapsulation with the previous nanoencapsulates.

Accordingly, a first aspect of the present invention relates to a multi-walled water-in-oil-in-water nanoencapsulate comprising internal water droplets which are dispersed within oil droplets, which are themselves dispersed in an external aqueous continuous phase; wherein: the internal water droplets comprise a chemical/biological active compound solubilized in the water and a first polyurethane-polyurea polymeric wall; the first polyurethane-polyurea polymeric wall is obtainable in a water-in-oil emulsification process by phase inversion through reaction at the interface of a polyisocyanate 1 with the amine groups of both (a) a first polyurethane/polyurea polymer (prepolymer 1), and (b) a water-soluble polyamine 1; the oil droplets comprise the internal water droplets dispersed therein and a second polyurethane-polyurea polymeric wall; the second polyurethane-polyurea polymeric wall is obtainable in an oil-in water emulsification process by phase inversion through its reaction at the interface of a polyamine 2 with the isocyanate groups of both (a) a second polyurethane/polyurea polymer (prepolymer 2) and (b) a polyisocyanate 2; both prepolymers, prepolymer 1 and prepolymer 2, have a main chain and side chains of different polarities; the main chains of both prepolymers comprises in its backbone urethane functional groups, urea functional groups, polyethylene oxide repeating units, disulfide bonds, amine rests, and optionally hydrophobic repeating units; and two end terminal functional groups; the two-end terminal functional groups of prepolymer 1 are amines terminal groups; and the two end terminal functional groups of prepolymer 2 are isocyanate terminal groups, wherein the prepolymer 2 is obtainable in situ from a prepolymer 3 which is the corresponding precursor with secondary amine end terminal groups instead of isocyanate groups.

A second aspect of the present invention relates to a process for the preparation of water-in-oil-in-water multi-walled polymeric nanoencapsulate comprising:
a) first carrying out a first encapsulation W/O by a process comprising the following steps: a1) Mixing a water-soluble chemical/biological active compound, a prepolymer 1), and a water-soluble polyamine 1, in a given volume of water; a2) Adding between 5 to 7-fold higher amount of an organic solvent immiscible in water to generate a first emulsion by means of a phase inversion; a3) Adding a polyisocyanate 1 soluble in the organic solvent selected for the emulsion to create the wall of the first encapsulation for the water-soluble chemical/biological agent through its stoichiometric reaction at the interface with the amines of the prepolymer 1 and the water-soluble polyamine 1;
b) Carrying out a second encapsulation O/W by a process comprising the following steps: b1) Adding to the previous encapsulation a prepolymer 3 which is the corresponding precursor of prepolymer 2 with secondary amine terminal groups instead of isocyanate groups; b2) Adding the mixture of step b1) over an equivalent excess of polyisocyanate with respect to the equivalent amount of the prepolymer 3 to form a prepolymer 2 *in situ*; b3) Adding the necessary amount of water to generate a second emulsion by means of a second phase inversion; b4) Adding a water-soluble polyamine 2 to create the wall of the second encapsulation through its reaction at the interface with the isocyanate from prepolymer 2 and the free polyisocyanate 2 added in excess;
c) Optionally, evaporating the organic solvents;
d) Optionally, dialyzing the water in oil in water emulsion of step c);
wherein prepolymer 1, prepolymer 2, and prepolymer 3 have independently a main chain and side chains of different polarities; the main chains of each of the prepolymers comprises in its backbone urethane functional groups, urea functional groups, polyethylene oxide repeating units, disulfide bonds, amine bonds, and optionally hydrophobic repeating units; and two end terminal functional groups; the two-end terminal functional groups of prepolymer 1 are secondary amine terminal groups; and the two end terminal functional groups of prepolymer 2 are isocyanate terminal groups; the two end terminal functional groups of prepolymer 3 are secondary amine terminal groups.

This technology can also be used to prepare structures that resemble liposomes. In this case the first nanoencapsulate is the same as described above, and then it is encapsulated in a superficial liposome structure. Thus, a third aspect of the present invention relates to an hybrid water-in-oil-in-water polyurethane/polyurea liposome comprising internal water droplets which are dispersed within larger oil droplets, wherein: a) the internal water droplets comprise a chemical/biological active compound solubilized in the water and a first polyurethane-polyurea polymeric wall; the first polyurethane-polyurea polymeric wall is obtainable in a water-in-oil emulsification process by phase inversion through reaction at the interface of a polyisocyanate 1 with the amine groups of both (a) a first polyurethane/polyurea polymer (prepolymer 1), and (b) a water-soluble polyamine 1; prepolymer 1 have a main chain and side chains of different polarities; the main chains comprise in its backbone urethane functional groups, urea functional groups, polyethylene oxide repeating units, disulfide bonds, amine bonds, and optionally hydrophobic repeating units; and two end terminal functional groups; the two-end terminal functional groups of prepolymer 1 are secondary amine terminal groups; and b) the oil droplets comprise the internal water droplets dispersed therein and an outer wall which resembles the structure of a liposome; the outer wall is obtainable in an oil-in-water emulsification through surface arrangement of di-palmitoyl-phosphatidylcholine and cholesterol.

A fourth aspect of the present invention relates to a process for the preparation of an hybrid water-in-oil-in-water polyurethane/polyurea liposome comprising internal water droplets which are dispersed within larger oil droplets as defined above which comprises the following steps: a) first carrying out a first encapsulation W/O by a process comprising the same steps as for the process for the preparation of the water-in-oil-in-water multi-walled polymeric nanoencapsulate described above; and then b) carrying out a second encapsulation O/W by a process comprising the following steps: b1) Adding to the previous encapsulation di-palmitoyl-phosphatidylcholine in a first suitable solvent and cholesterol in a second suitable solvent; b2) Adding the necessary amount of water to generate a second emulsion by means of a second phase inversion and create and outer wall through physical rearrangement of di-palmitoyl-phosphatidylcholine and cholesterol; c) Optionally, evaporating the organic solvents; and d) Optionally, dialyzing the water in oil in water emulsion of step c).

A fifth aspect of the present invention relates to a composition comprising a nanoencapsulate or a liposome as defined above together with carriers. Finally, a sixth aspect of the present invention relates to a cosmetic or pharmaceutical composition comprising a nanoencapsulate or a liposome as defined above, together with cosmetically or pharmaceutically acceptable excipients and/or carriers.

### Brief Description of Drawings

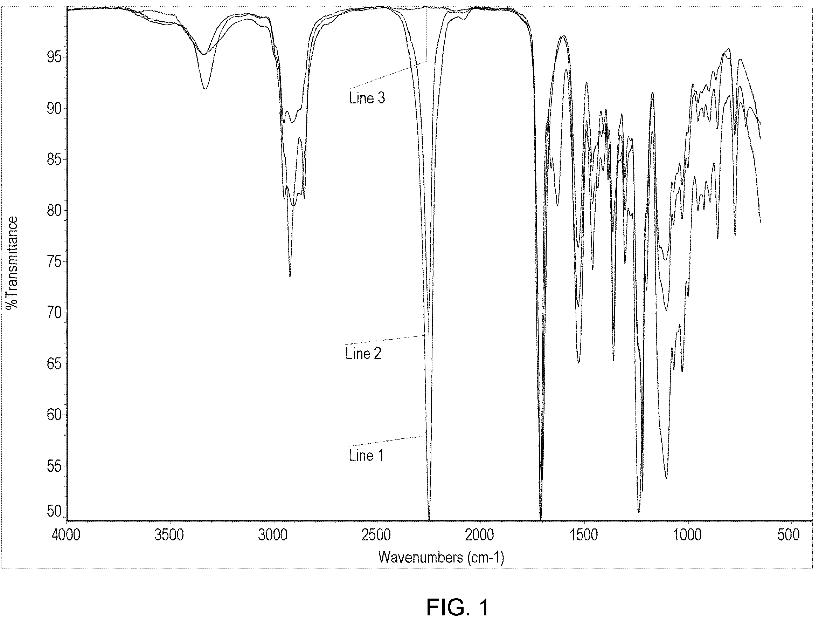

FIG. 1 shows the IR spectra of the formation of polymer P1. The line 1 corresponds to the first sample recorded, at the start of the reaction (sharp NCO asymmetric stretching band at 2252 cm⁻¹). The line 2 shows the end of the first step of the synthesis involving the reaction between the diols and the diisocyanate (intensity of the NCO stretching band decreased significantly, intensities of the CO stretching band at 1719 cm⁻¹, the CN stretching band at 1537 cm⁻¹, the NCOO/COC asymmetric stretching band at 1240 cm⁻¹ increased). The line 3 shows the effect of adding the diamine during the second step of the synthesis (NCO stretching band at 2252 cm⁻¹ disappeared instantaneously, simultaneously, other characteristic bands appeared or changed, such as a new stretching band at 1634 cm⁻¹, which was associated to the carbonyl of urea bonds and a new wagging band at 908 cm⁻¹ corresponding to the free secondary amine).

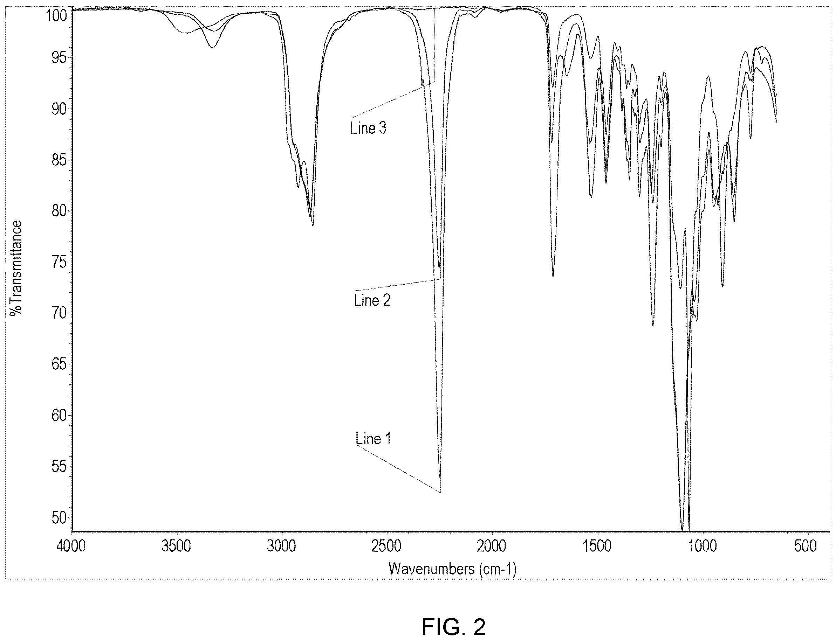

FIG. 2 shows the IR spectra of the formation of polymer P3. As in FIG.1, the line 1 corresponds to the first sample recorded, at the start of the reaction (sharp NCO asymmetric stretching band at 2252 cm⁻¹). The line 2 shows the end of the first step of the synthesis involving the reaction between the diols and the diisocyanate (intensity of the NCO stretching band decreased significantly, intensities of the CO stretching band at 1719 cm⁻¹, the CN stretching band at 1537 cm⁻¹, the NCOO/COC asymmetric stretching band at 1240 cm⁻¹ increased). The line 3 shows the effect of adding the diamine during the second step of the synthesis (NCO stretching band at 2252 cm⁻¹ disappeared instantaneously, simultaneously, other characteristic bands appeared or changed, such as a new stretching band at 1634 cm⁻¹and a new wagging band at 908 cm⁻¹).

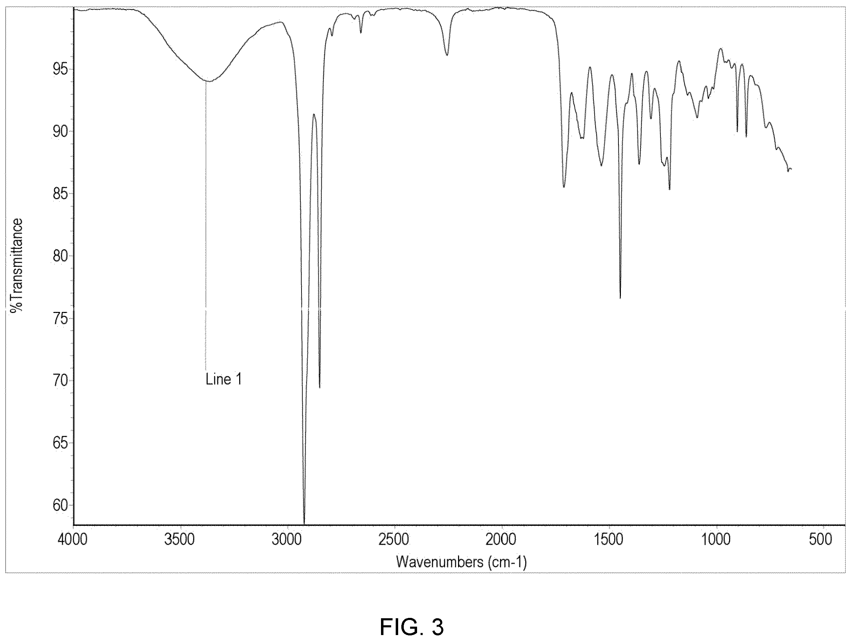

FIG. 3 shows the IR spectra of the W/O encapsulation reaction.

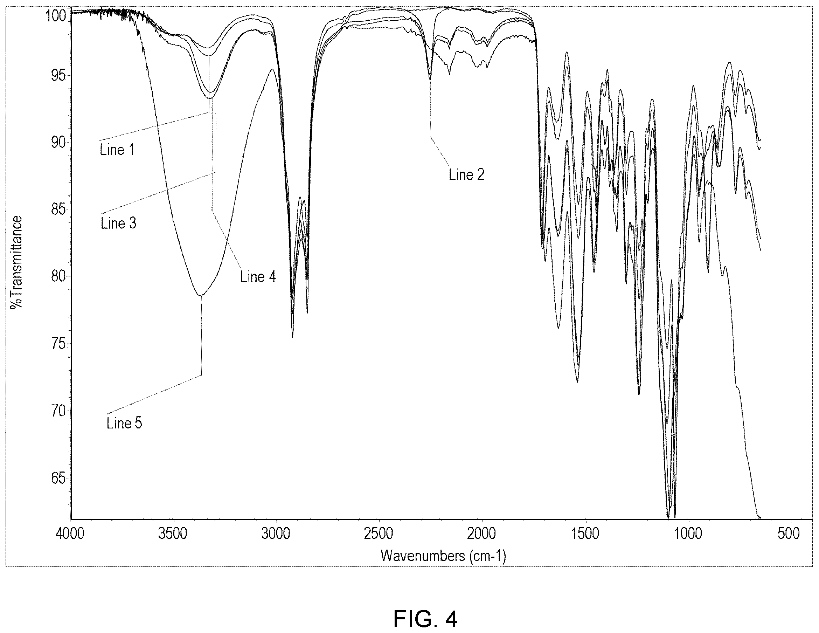

FIG. 4 shows the W/O/W encapsulation reaction. The line 1 represents the sample after the P1+NCs1 mixing. The line 2 corresponds to the P1+NCs1 mixture added over determinate diisocyanate amount to reactivate the polymer and convert to an NCO-reactive entity. The line 3 shows the urea formation by the subsequent reaction between the activated polymer and L-lysine sodium salt that had been added (decrease on the intensity of the NCO stretching band at 2255 cm⁻¹, concomitantly with an increase of the carbonyl and CN stretching bands). The line 4 shows that NCO stretching band is maintained where is represented the phase inversion with MilliQ water. The line 5 shows the urea formation after adding the triamine (NCO stretching band instantaneously disappeared and the urea-associated bands increased their intensity as a result of the rapid reaction between remaining NCO groups and this polyamine).
FIG. 5 shows Z-pot vs pH in each sample of carboplatin nanoencapsulates of Example 7.
FIG. 6 shows a graphic representation of coumarin-derivative dye expression on DCs over time (6h and 24h), with a negative control where DCs were exposed to JB-H2O-PPAH-PP22-25. The graphic depicts percentages of coumarin-positive DCs from n=4 independent experiments.
FIG. 7 shows how the corrected results maintain the internalization ratio of the different NCs concentrations. coumarin-loaded nanoparticles corrected internalization. 4° positive percentages have been subtracted from 37°C ones. Graphic depicts n=1 experiment.
FIG. 8 shows R848-loaded nanoparticles immunostimulatory effect on DCs at different concentrations after 48h incubation. A) Percentages of CD80-FITC positive DCs from n=3 independent experiments. B) Percentages of CD83-PE positive DCs from n=2 independent experiments.
FIG. 9 shows R848-loaded nanoparticles cytotoxicity effect on DCs at different concentrations after 48h incubation. The graphic depicts percentages of 7-AAD-PerCp5.5 positive cells from n=1 independent experiment.
FIG. 10 a) and b) show the selection of proper cells for the flow cytometry fluorescence analysis.
FIG. 11 a) shows the number of fluorescence positive cells incubated with non-loaded nanocapsules and FIG. 11 b) shows the number of fluorescence positive cells incubated with ICG-loaded nanocapsules.
FIG. 12 shows a fluorescence microscopy image registered in a Thunder Microscope. Qualitative analysis of intracellular localization of fluorophore-loaded nanocapsules.
FIG. 13A shows the intravenous administration of 160 µl, 40 µM of ICG free, on BALB/C mice group (n=4 mouse).
FIG. 13B shows the intravenous administration of 160 µl, 40 µM of ICG in 30 mg/mL of cationic ICG-NCs, on BALB/C mice group (n=4 mouse).
FIG. 13C shows the intravenous administration of 160 µl, 40 µM of ICG in 30 mg/mL of amphoteric ICG-NCs, on BALB/C mice group (n=4 mouse).
FiG. 14 shows in vivo imaging of systemic biodistribution of ICG labelled nanocapsules in NSG mice:
   A) A375 challenged NSG mice treated with vehicle (PBS) (n=3/group).
   B) A375 challenged NSG mice treated with free ICG (n=3/group).
   C) A375 challenged NSG mice treat with amphoteric ICG-NCs (n=3/group). Locations for tumor cells injections have been indicated with a red circle.
FIG. 15 shows the growth curves and tumour growth of subcutaneously implanted cancer cells in C57BL/6 mice after different treatments. B16.F0 (7×10⁴) cells were injected subcutaneously into C57BL/6 mice. Tumour growth (area in mm²) and weight (g) were measured every other day and at sacrifice, respectively. Data are presented as mean ± SEM.
FIG. 16 shows the immunophenotypical analysis of spleens from C57BL/6 mice challenged with B16.F0 (7×10⁴) cells and i.v. or s.c treated with PBS, R484, cationic R848-NCs or amphoteric R848-NCs nanocapsules. A) Total lymphoid cell numbers. B) NK, CD8+ T cells and CD4+ T cells.

### Detailed description of the invention

### Definitions

All terms as used herein in this application, unless otherwise stated, shall be understood in their ordinary meaning as known in the art. Other more specific definitions for certain terms as used in the present application are as set forth below and are intended to apply throughout the description and claims.

The term "about" or "around" or "approximately" as used herein refers to a range of values ± 10% of a specified value. For example, the expression "about 10" or "around 10" includes ± 10% of 10, i.e., from 9 to 11.

The expression "one or more" as used herein refers to the fact that there may be one or more of the entities referred to, in particular 1, 2, 3 or 4.

As used herein, the term "optional" means that the element or step to which this term refers, may or may not be present.

As used herein, the terms "drug" and "therapeutic agent" are used interchangeably.

Where in the present invention a numerical interval is used, this includes the values of the extremes of the interval. In particular, as used herein, the term "comprised between" to refer to a range of values including the end points of the range.

The word "comprise" for the purposes of the present invention encompasses the case of "consisting of".

Unless otherwise stated, all percentages mentioned herein are expressed in weight with respect to the total weight of the product, provided that the sum of the amounts of the components is equal to 100%.

The term "phase inversion" refers to a phenomenon that occurs when agitated oil in water, in presence of a self-emulsifiable polymer, reverts to a water in oil and vice versa. Emulsification via phase inversion is widely used in fabrication of cosmetic products, pharmaceutical products (e.g., vesicles for drug delivery), foodstuff and detergents. Phase inversion process leads to the formation of finely dispersed droplets in a continuous phase.

The term "at room temperature" means a temperature that can be comprised in the range of 18-30 °C, generally 20-25 °C.

As used herein, the term "side chains of different polarities" refers to side chains with different functional groups or same groups introduced in a different ratio, which varies the physicochemical properties of the polymer chain.

As used herein, the term "hydrophobic" refers to a polymer that lacks affinity for water That is, it tends to repel water, to not dissolve in, mix with or be wetted by water or to do so only to a very limited degree and to not absorb water or, again, to do so only to a very limited degree.

As used herein, the term "lipophilic" or the term "liposoluble" refers to the ability of a compound to dissolve in fats, oils, lipids, and non-polar solvents such as hexane or toluene.

As used herein, "hydrophilic" refers to a compound that is attracted to water molecules and tends to be dissolved by water.

As used herein, the term "amphipathic lipid" (also termed sometimes as amphiphilic) refers to any suitable material wherein the hydrophobic portion of the lipid material orients into a hydrophobic phase, while a hydrophilic portion orients toward the aqueous phase.

As used herein, the term " hydrophilic/lipophilic balance" of a compound is a measure of the degree to which it is hydrophilic or lipophilic, determined by calculating values for the different regions of the molecule, as described by Davies (see "A quantitative kinetic theory of emulsion type, I. Physical chemistry of the emulsifying agent" , Gas/Liquid and Liquid/Liquid Interface, Proceedings of the International Congress of Surface Activity, pp. 426-38). In particular, the method is based on a value based on the chemical groups of the molecule.

The term "encapsulation" can refer to a formulation that provides a compound with fully crosslinked encapsulation, partially crosslinked encapsulation, or combinations thereof.

As used herein, the term aliphatic compounds refer to hydrocarbon compounds which are non-aromatic hydrocarbons. Aliphatic compounds can be saturated, like hexane, or unsaturated, like hexene and hexine.

As used herein a non-polar solvent relate to compounds that have low dielectric constants and are not miscible with water.

The first aspect of the present invention relates to a multi-walled water-in-oil-in-water nanoencapsulate comprising internal water droplets which are dispersed within oil (or water immiscible phase) droplets, which are themselves dispersed in an external aqueous continuous phase with the following features:
(1) The internal water droplets comprise a chemical/biological active compound solubilized in the water and a first polyurethane-polyurea polymeric wall; the first polyurethane-polyurea polymeric wall is obtainable in a water-in-oil emulsification process by phase inversion through reaction at the interface of a polyisocyanate 1 with the amine groups of both (a) a first polyurethane/polyurea polymer (prepolymer 1), and (b) a water-soluble polyamine 1;
(2) The oil droplets are considered the "organic phase or dispersed phase for the second emulsion and comprise the internal water droplets dispersed therein and a second polyurethane-polyurea polymeric wall; the second polyurethane-polyurea polymeric wall is obtainable in an oil-in water emulsification process by phase inversion through its reaction at the interface of a polyamine 2 with the isocyanate groups of both (a) a second polyurethane/polyurea polymer (prepolymer 2) and (b) a polyisocyanate 2;
both prepolymers, prepolymer 1 and prepolymer 2, have a main chain and side chains of different polarities; the main chains of both prepolymers comprises in its backbone urethane functional groups, urea functional groups, polyethylene oxide repeating units, disulfide bonds, amine bonds, and optionally hydrophobic repeating units; and two end terminal functional groups; the two-end terminal functional groups of prepolymer 1 are amine terminal groups; and the two end terminal functional groups of prepolymer 2 are isocyanate terminal groups, wherein the prepolymer 2 is obtainable in situ from a prepolymer 3 which is the corresponding precursor with secondary amine end terminal groups instead of isocyanate groups.

The internal water droplets are dispersed within oil droplets, being the oil droplets larger than the internal water droplets.

In a particular embodiment, the amine terminal groups of prepolymer 1 are primary amine terminal groups. In another particular embodiment, the amine terminal groups of prepolymer 1 are secondary amine groups.

Examples of hydrophobic repeating units are C36 aliphatic dimer diol (Pripol 2033),N-octadecylpropane-1,3-diamine (Genamin TAP 100D or Duomeen T), and (1E,19E)-10,11-dioctylicosa-1,19-diene-1,20-diamine (Priamine 1074).

In a particular embodiment, the main chain of any of the prepolymers 1 or 2 comprises hydrophobic repeating units.

The side chains of different polarities may be for instance alkyl chains, alkyl amine chains, or alkyl ether chains, among others. The alkyl chain may be for instance a (C₂-C₁₂)-alkyl chain, in a particular embodiment, the alkyl chain is a (C₃-C₁₀)-alkyl chain, in another particular embodiment, the alkyl chain is a (C₄-C₈)-alkyl chain. The alkyl amine may be for instance a (C₂-C₁₂)-alkyl amine, in a particular embodiment, the alkyl is a (C₃-C₁₀)-alkyl amine, in another particular embodiment, the alkyl is a (C₄-C₈)-alkyl amine. They can be primary, secondary, or tertiary amines. The alkyl ether may be for instance a (C₂-C₁₂)-alkyl ether, in a particular embodiment, the alkyl is a (C₃-C₁₀)-alkyl ether, in another particular embodiment, the alkyl is a (C₄-C₈)-alkyl ether. In a particular embodiment, the alkyl ether is a polyethyleneglycol ether, in particular having a (C₂-C₁₂) carbon atoms.

In another particular embodiment, a polyalcohol or polyamine carbohydrate is introduced in the polymer backbone, in particular having biotarget or biodegradable groups.

In a particular embodiment, in combination with any of the embodiments above or below, the multi-walled water-in-oil-in-water nanoencapsulate defined above is a double-walled water-in-oil-in-water nanoencapsulate.

In the first encapsulation, the first emulsion can be generated by means of a phase inversion mediated by slowly addition with high agitation at room temperature of an organic solvent immiscible in water and by the emulsifying capacity of the pre-designed amphiphilic polymer at the moment when this solvent is at a higher concentration than the water in the reaction vessel.

The second inversion is carried out when the concentration of water is greater than that of the organic phase in the reaction vessel during the second encapsulation step.

In another particular embodiment, in combination with any of the embodiments above or below, the multi-walled water-in-oil-in-water nanoencapsulate as defined above is that having a hydrodynamic diameter comprised in the range from 20 to 50 nm, preferably in the range from 25 to 40 nm. The hydrodynamic diameter is measured with a Zetasizer Nano ZS90 (Dynamic Light Scattering) in a concentration between 0.05 to 0.1 % of solid content.

Hydrodynamic Diameter (D_{H}) is the diameter of a hard sphere that diffuses at the same speed as the particle or molecule being measured. The hydrodynamic diameter depends not only on the size of the particle "core", but also on any surface structure, as well as the type and concentration of any ions in the medium.

The prepolymers composition ensures that the nanodroplets remain stable during emulsification and do not grow to a large size. Prepolymer 1 and the prepolymer 2 are self-emulsifiable reactive polymers, they behave as dispersants and self-emulsify the emulsion, while at the same time, are reactive, which means that they intervene in the encapsulation forming the wall of the nanoencapsulate. The self-emulsifying properties of the prepolymers avoid the addition of external surfactants and the need of high shear (between 400-500 rpm) stirring during emulsification. This is advantageous because some additives, such as surfactants, are generally difficult to remove from the final product. When the product is either food, cosmetics, or pharmaceutical, this issue becomes more serious since the remaining emulsifiers can induce contact allergies or hypersensitivities in the consumers. Besides, it is advantageous to avoid the need of high speed stirring because these requirements are difficult to achieve in large-scale productions due to technical limitations.

The process for preparing the prepolymers 1 and 2 is an easily scalable process to prepare multifunctional polyurethane/polyurea prepolymers. There can be certain degree of functionality. A higher degree of functionality can be achieved by using prepolymers having a higher degree of functionality or by adding such functionality in the preparation of the multiwalled functionalizable polyurethane-polyurea nanoencapsulates. In this regard, the chemical versatility behind this process allows the incorporation of a broad range of potential functionalities, such as biodegradable bonds, for example the disulfide bonds that are biodegradable under reductive conditions.

It may also comprise carbohydrates-based moieties, targeting ligands, charged monomers, and dangling chains of different polarities. Finally, these functionalities may also be peptides, esters, ethers, etc.

Besides, the designs of the prepolymers can be modified and provided with varied biological functionalities to provide greater biodegradability, selective release under certain conditions of the extra or intracellular medium, modification of its surface charge according to the medium, selective accumulation in characteristic areas of the organism or in target tissues, etc.

In a particular embodiment, the multi-walled water-in-oil-in water nanoencapsulate as defined above is that where the polyurethane-polyurea polymeric walls are independently functionalized with a group selected from a diol ester group, a diol fluor containing group, a diamine fluor containing group, a polyalcohol carbohydrate group, a polyamine carbohydrate group, a peptide group, a diol ether group, a targeting ligand, a bioligand, and a charged monomer. The functionalization is carried out through polyurethane/polyurea formation by the reaction of polyalcohol and polyamines against isocyanate groups.

The prepolymers 1 and 2 can also be defined by its preparation process.

Prepolymer 1 can be obtainable by a process comprising reacting: a1) a polyalcohol comprising disulfide bonds; b1) a polyalcohol; c1) a polyamine; and d1) a polyisocyanate.

The polyalcohol comprising disulfide bonds a1) can be for example a dihydroxy (C₂-C₆)alky disulfide. In a particular embodiment, the polyalcohol comprising disulfide bonds a) is 2.2'- dihydroxyethyl disulfide (DEDS).

The polyalcohol b1) can be a polyethylene glycol having a molecular weight MW of from 1000-3000 (like YMER N120, a linear difunctional polyethylene glycol monomethyl). The molecular weight can be measured by Size Exclusion Chromatography. In a particular embodiment, polyethylene glycol having a molecular weight MW of from 1000-3000 are commercial or known compounds. In another particular embodiment, the polyethylene glycol has a molecular weight MW of 1000. In another particular embodiment, the polyethylene glycol is diol polyethylene glycol. The polyalcohol can also be a fatty diol such as a dimer diol, for example Pripol 2033 ^{®} that is a dimer linoleic diol.

The polyamine c1) is a (C₁₀-C₄₀) aliphatic polyamine, i.e., it is a hydrophobic compound; the polyamine can be linear or branched. In another particular embodiment the polyamine c1) is a linear (C₁₀-C₄₀)alkyl polyamine. This (C₁₀-C₄₀)alkyl polyamines can have primary or secondary groups. In another particular embodiment the linear or branched (C₁₀-C₄₀)alkyl polyamines have an (C₁₀-C₂₀) alkyl chain. This aliphatic chain contributes to the micellar morphology and stabilization of the encapsulated molecule. In a particular embodiment the (C₁₀-C₄₀) aliphatic polyamine is a (C₁₀-C₂₅) aliphatic polyamine.

In another particular embodiment the C₁₀-C₄₀) alkyl polyamines is selected from the group consisting of 1,3-diamino-N-octadecyl propane, N-(3-dimethylaminopropyl)-N,N'-diisopropanolamine, (Z)-N-9-octadecenylpropane-1,3-diamine, (Z)-octadec-9-enylamine, N-tetradecylpropane-1,3-diamine, N-dodecylpropane-1,3-diamine, N-[(10Z)-heptadec-10-en-1-yl]propane-1,3-diamine or N-[(9Z)-hexadec-9-en-1-yl]propane-1,3-diamine. In another particular embodiment, the (C₁₀-C₄₀)alkylamine is 1,3-diamino-N-octadecyl propane.

The polyisocyanate d1) can be for example an aliphatic diisocyanate or an alkyl aromatic diisocyanate. In a particular embodiment, the polyisocyanate is selected from the group consisting of isophorone diisocyanate (IPDI), methyl-xylylene diisocyanate (MXDI), Tetramethylxylene diisocyanate (TMXDI) hexamethylene diisocyanate (HDI), trimethylhexamethylene diisocyanate (TMDI), Dicyclohexylmethane-4,4'-diisocyanate (Desmodur W^{®} from Covestro) or difunctional prepolymers from previous ones. In another particular embodiment, the polyisocyanate is isophorone diisocyanate which is specially preferred because its reduced toxicity due to the aliphatic structure and relatively high vapor pressure and because the two NCO groups act with different reactivity, which contributes to an improved polyaddition control.

In a particular embodiment, the equivalent ratio of the starting components of prepolymer 1 is as follows:
Polyisocyanate d1) / polyalcohol a1 (disulfide bonds) in a range from 1:0.3 to 1:0.6;
Polyisocyanate d1) / polyalcohol b1 (polyethylene glycol) in a range from 1:0.05 to 1:0.15; and
Polyisocyanate d1) / polyamine c1 (hydrophobic tailored) in a range from 1:0.2 to 1:0.8.

In another particular embodiment, the equivalent ratio of the starting components of prepolymer 1 is as follows:
Polyisocyanate d1) /polyalcohol a1 (disulfide bonds) 1:0.45;
Polyisocyanate d1) / polyalcohol b1 (polyethylene glycol) 1:0.09; and
Polyisocyanate d1) / polyamine c1 (hydrophobic tailored): 1:0.55

Prepolymer 2 is obtainable *in situ* when forming the second polyurethane-polyurea polymeric wall from the corresponding amino precursor prepolymer (prepolymer 3).

Prepolymer 3 can be obtainable by a process comprising reacting: a2) a polyalcohol comprising disulfide bonds; b2) a polyalcohol; c2) a polyamine d2) a diol-polyamine; and e2) a polyisocyanate.

The polyalcohol comprising disulfide bonds a2) is independently selected from the same group as polyalcohol a1).

The polyalcohol b2) is independently selected from the same group as polyalcohol b1).

The polyamine c2) is independently selected from the group as polyamine c1).

The diol-polyamine d2) is selected from the group consisting of tertiary (C₈-C₂₀)alkyl diamines which include two alcoholic positions. Examples include N(3-dimethylaminopropyl)-N,N-diisopropanolamine.

The polyisocyanate e2) is independently selected from the same group as polyisocyanate d1).

Prepolymers 1 and 3 can be prepared in the presence of solvents. A suitable solvent for the preparation of prepolymer 1 is for instance acetone. A suitable solvent for the preparation of prepolymer 3 is for instance tetrahydrofuran.

In a particular embodiment, the equivalent ratio of the starting components of prepolymer 3 is as follows:
In a particular embodiment, the equivalent ratio of the starting components of prepolymer 3 is as follows:
Polyisocyanate e2) / polyalcohol a2 (disulfide bonds) in a range from 1:0.05 to 1:0.2;
Polyisocyanate e2) / polyalcohol b2 (polyethylene glycol) in a range from 1:0.2 to 1:0.6;
Polyisocyanate e2) / polyamine c2 (hydrophobic tailored) in a range from 1: 0.2 to 1:0.8, and
Polyisocyanate e2) / diol-polyamine d2 in a range from 1:0.05 to 1:0.3.

In another particular embodiment, the equivalent ratio of the starting components of prepolymer 3 is as follows:
Polyisocyanate e2) / polyalcohol a2 (disulfide bonds) 1:0.16
Polyisocyanate e2) / polyalcohol b2 (polyethylene glycol) 1:0.31
Polyisocyanate e2) / polyamine c2 (hydrophobic tailored): 1:0.48
Polyisocyanate e2) / polyamine d2 (diol-diamine): 1:0.12

The prepolymers containing such functionalities create ordered shells due to their HLB and self-stratification at the oil-water interface or water-oil interface, leading to multiwalled structures. The HLB of these prepolymers can also be increased or decreased to control the particle size of the final nano-encapsulation.

In a particular embodiment, in combination with any of the embodiments above or below, the prepolymer 1 has a HLB value equal to or lower than 10.

In another particular embodiment, in combination with any of the embodiments above or below, prepolymer 3 has a HLB value higher than 10. In another particular embodiment, the HLB is equal to or higher than 15.

In a particular embodiment of the first aspect of the present invention, in combination with any of the embodiments above or below, in the preparation of the first polyurethane-polyurea polymeric wall, the water-soluble polyamine 1 is selected from the group consisting of diamine, triamine, tetraamine, and pentaamine. The water soluble polyamine 1 can be an aliphatic or an alkyl-aromatic polyamine. In a particular embodiment, the polyamine is an alkyl-aromatic amine. In another particular embodiment the aromatic amine is methyl xylylene diamine. In another particular embodiment, the polyamine is an aliphatic amine. In another particular embodiment, in combination with any of the embodiments above or below, the polyamine 1 is selected from the group consisting of ethylene diamine (EDA), diethylenetriamine (DETA), triethylenetetramine (TETA), L-lysine, guanidine, methyl xylylene diamine (MXDA), a sulphonated polyamine, and a carboxylated polyamine. In another particular embodiment, in combination with any of the embodiments above or below, the polyamine is selected from the group consisting of ethylene diamine (EDA), diethylenetriamine (DETA), triethylenetetramine (TETA), and L-lysine, and combinations thereof.

In a particular embodiment of the first aspect of the present invention, in combination with any of the embodiments above or below, in the preparation of the first polyurethane-polyurea polymeric wall, the polyisocyanate 1 is a polyaliphatic isocyanate which ensures good biocompatibility and reduced toxicity of biodegraded products. In another particular embodiment, in combination with any of the embodiments above or below, the polyaliphatic isocyanate is selected from the diisocyanates group consisting of isophorone diisocyanate (IPDI), methyl-xylylene diisocyanate (MXDI), Tetramethylxylene diisocyanate (TMXDI) hexamethylene diisocyanate (HDI), trimethylhexamethylene diisocyanate (TMDI), Dicyclohexylmethane-4,4'-diisocyanate (Desmodur W^{®} from Covestro), and difunctional NCO-terminal polyurethane-based prepolymers obtained from previous ones. In another particular embodiment, in combination with any of the embodiments above or below, the polyaliphatic isocyanate is selected from the polyisocyanates group consisting of trimethylol propane-adduct of xylylene diisocyanate (Takenate D110-N from Mitsui Chemicals), 1,3,5-tris(5-isocyanatopentyl)-1,3,5-triazinane-2,4,6-trione and 2-methylpropyl N-(5-isocyanatopentyl)-N-(5-isocyanatopentylcarbamoyl)carbamate (Stabio N376 from Mitsui Chemicals), Aliphatic polyisocyanate HDI uretdione (like Desmodur N-3400 and N-3600 from Covestro), Hydrophilic aliphatic polyisocyanate based on hexamethylene diisocyanate (like Bayhydur XP2655 and Bayhydur 3100 from Covestro), and polyfunctional NCO-terminated prepolymers.

In a particular embodiment of the first aspect of the present invention, in combination with any of the embodiments above or below, in the preparation of the second polyurethane-polyurea polymeric wall, the polyamine 2 is independently selected from the same group as polyamine 1 defined above, including mixtures thereof. In a particular embodiment in combination with any of the embodiments above or below, the polyamine 2 is a mixture of DETA and L-lysine. In a particular embodiment in combination with any of the embodiments above or below, the polyamine 2 is a polyamine carbohydrate. In a particular embodiment in combination with any of the embodiments above or below, the polyamine 2 is chitobiose or chitosan. The use of L-lysine as an extender or precrosslinker modulated the size and the surface characteristics of the micelles, reaching very small entities that finally were stabilized by crosslinking with DETA or another type of polyamine derivative.

In a particular embodiment of the first aspect of the present invention, in combination with any of the embodiments above or below, in the preparation of the second polyurethane-polyurea polymeric wall, the polyisocyanate 2 is independently selected from the same group as polyisocyanate 1 defined above.

The prepolymers of the present invention can be functionalized both chemically and with bioligands in a versatile and easy way. The functionalization can be carried out as disclosed in EP2992953A2 for the outer wall. The functionalization of the internal wall can be carried out analogously. This may allow that the nanocapsule can be directed to a certain place of the organism, and when the outer wall disappears in such place by having the internal wall also functionalized the nanocapsule can be directed to other more internal sites of the cell such as organelles or the lymphatic system.

In a particular embodiment of the first aspect of the present invention, in combination with any of the embodiments above or below, in the preparation of the polyurethane-polyurea polymeric walls, a diol ester group is introduced to polyurethane backbone, through direct reaction of alcohols and isocyanates in the synthesis of prepolymer 1 or prepolymer 3, to regulate the specific release in presence of determined enzymes (mostly esterases) or using localized heating (in case of low-melting point esters). In another particular embodiment, one or both of previously described walls contain, in the preparation of the polyurethane-polyurea polymeric walls, a butanediol adipate or polybutylene succinate moiety which is introduced to polyurethane backbone, for a specific release under ester degradation conditions.

In a particular embodiment of the first aspect of the present invention, in combination with any of the embodiments above or below, in the preparation of the polyurethane-polyurea polymeric walls, a diol or diamine fluor containing group is introduced to polyurethane backbone in the synthesis of prepolymer 1 or prepolymer 3 to reach oleophobic or superhydrophobic characteristics. In another particular embodiment, one or both of previously described walls contain a perfluoropolyether (Fluorolink E10H) to obtain high-performance coatings.

In a particular embodiment of the first aspect of the present invention, in combination with any of the embodiments above or below, in the preparation of the polyurethane-polyurea polymeric walls, a carbohydrate group is introduced to polyurethane backbone, through amine or alcohol reaction with free isocyanate groups during the synthesis of polymer 1 or in situ in the NCs2 synthesis process (in the case of water soluble aminated-carbohydrate), to enhance biocompatibility/biodegradability, increase the RES-evading behavior, or specific organ/tissue targeting. In another particular embodiment, one or both of previously described walls contain xylitol, erythritol or chitobiose, increasing the biodegradability and biocompatibility of the overall nanocapsule.

In a particular embodiment, of the first aspect of the present invention, in combination with any of the embodiments above or below, in the preparation of the polyurethane-polyurea polymeric walls a tumor cell director is introduced to polyurethane backbone in the synthesis of nanocapsules. The functionalizing group have two main functional groups, in this way it remains integrated in the main chain of the prepolymer. The functional group can be a monoclonal antibody or include a monoclonal antibody.

In another particular embodiment, of the first aspect of the present invention, in combination with any of the embodiments above or below, in the preparation of the polyurethane-polyurea polymeric walls a peptide is introduced to polyurethane backbone. This peptide may be for example in the outer wall of the nanocapsule and may be able to interact with overexpressed proteins outside the tumor cells. The peptide may be for example c-(RGDfK). Folic acid may be incorporated in the same way as c-(RGDfK). It may interact with folate receptor proteins that are overexpressed in numerous cancer cells.

When the functionalizing element does not have two main functional groups, it can be first reacted with a linker such as for example a di- or polyisocyanate. This way, the link between the functional element and the linker makes it possible to obtain the functional group indicated above. As a way of example, the peptide can be reacted with a di- or polyisocyanate (such as for example Bayhydur 3100 ^{®}) and the reaction product can be added to the organic phase of the second encapsulation.

In another particular embodiment of the first aspect of the present invention, in combination with any of the embodiments above or below, in the preparation of the polyurethane-polyurea polymeric walls, a diol ether group is introduced to polyurethane backbone during the synthesis of prepolymer 1 or prepolymer 3.

In another particular embodiment, in combination with any of the embodiments above or below, the multi-walled water-in-oil-in-water nanoencapsulate as defined above is that wherein the prepolymer 2 is obtainable *in situ* from a precursor of prepolymer 3 with the same structure than prepolymer 2 but with secondary amino end terminal groups.

The polyurethane/polyurea composition of prepolymer 3 have a HLB value equal to or lower than prepolymer 1 (theoretical values calculated using Davies method mentioned above).

In a particular embodiment, the multi-walled water-in-oil-in-water nanoencapsulate as defined above is that where the chemical/biological active compound solubilized in the water is selected from the group consisting of pharmaceutical active ingredients, peptides, antibodies, vitamin C, pH regulators, oligonucleotides, carbohydrates, water-soluble fluorophores, hydrosoluble metallodrugs, and hydrosoluble oxygen barrier compounds (for applications in gas barrier coatings).

In another particular embodiment, the multi-walled water-in-oil-in-water nanoencapsulate as defined above is that which has hydrosoluble peptides encapsulated in the central water core.

In another particular embodiment, the multi-walled water-in-oil-in-water nanoencapsulate as defined above is that which has Bac7, TAT, Vancomycin or Polymyxin B encapsulated in the central water core. Bac7 and TAT are known peptides useful as antibiotics.

In another particular embodiment, the multi-walled water-in-oil-in-water nanoencapsulate as defined above is that where the chemical/biological active compound solubilized in the water (hydrophilic drug) is a TLR7/8 agonist to activate the immune system against melanoma. In another particular embodiment, the multi-walled water-in-oil-in-water nanoencapsulate as defined above is that where the chemical/biological active compound solubilized in the water is Resiquimod (R848) or Imiquimod. When the drug is Resiquimod or Imiquimod the nanoencapsulates of the present invention are for use in immunotherapy.

In another particular embodiment, the multi-walled water-in-oil-in-water nanoencapsulate as defined above is that where the chemical/biological active compound solubilized in the water (hydrophilic drug) is a chemotherapeutic agent selected from Cisplatin, Carboplatin, Oxaliplatin, Gemcitabine and Etoposide. When the drug is a chemotherapeutic agent, the nanoencapsulates of the present invention are for use in chemotherapy.

Other examples of drugs that can be encapsulated are the following: Remdesivir, in which case the nanoencapsulate containing it is for use as antiviral; a cocktail of Gemcitabine + Paclitaxel, in which case the nanoencapsulate containing them is for use in chemotherapy; Resiquimod + Irinotecan, Cisplatin + Resiquimod, or Paclitaxel + Resiquimod, in which cases the nanoencapsulate containing them is for use is chemotherapy and immunotherapy; Meropenem, Ertapenem, Tobramycin or Ciprofloxacin in which case the nanoencapsulates are for use as antibiotic.

In another particular embodiment, the multi-walled water-in-oil-in-water nanoencapsulate as defined above is that which has oligonucleotides such as DNA or RNA encapsulated in the central water core. The nanoencapsulates of oligonucleotides are for use in genetic therapy. The encapsulation of these compounds with the multi-walled water-in-oil-in-water nanoencapsulate is advantageous because these compounds generally used in gene therapy are usually very sensitive to the environment (temperature, air, etc..) and denature or degrade easily, causing even their inactivation.

In another particular embodiment, the multi-walled water-in-oil-in-water nanoencapsulate as defined above is that which has a peptide, in which case the nanoencapsulates are for use in different medical fields (oncology, antimicrobials, regenerative medicine, etc).

In another particular embodiment, the multi-walled water-in-oil-in-water nanoencapsulate as defined above is that which has an antibody, in particular a monoclonal antibody, for example anti-Her 2, in which case the nanoencapsulates are for use in the treatment of breast cancer.

In another particular embodiment, the multi-walled water-in-oil-in-water nanoencapsulate as defined above is that which has water-soluble UV-visible, NIR or Far-red fluorophores, encapsulated in the central water core. In another particular embodiment, the hydrosoluble fluorophore is 4',6-Diamidine-2'-phenylindole dihydrochloride (DAPI). In another particular embodiment, the hydrosoluble fluorophore is propidium iodide. In another particular embodiment, the hydrosoluble fluorophore is Indocyanine Green. In another particular embodiment, the hydrosoluble fluorophore is Dil. The nanocapsule comprising an hydrosoluble fluorophore are for use in bioimaging, in particular for monitoring nanocapsules biological pathway and for differentiation on free and encapsulated accumulation.

In another particular embodiment, the multi-walled water-in-oil-in-water nanoencapsulate as defined above is that which has hydrosoluble metallodrugs encapsulated in the central water core. Examples of these hydrosoluble metal pharmaceuticals are cisplatin, carboplatin, Hydrosoluble Ruthenium and Iridium-based compounds and derivatives.

In another particular embodiment, the multi-walled water-in-oil-in-water nanoencapsulate as defined above is that which encapsulate saline buffers in the central water core. Example of saline buffers are ascorbic acid, carbonates, or other buffers, which allow to regulate the pH in extra or intracellular areas of unregulated activity. The nanoencapsulates with saline buffers are used in cosmetic and pharmaceutical products. In another particular embodiment, the multi-walled water-in-oil-in-water nanoencapsulate as defined above is that which encapsulate hydrosoluble oxygen barrier compounds (for application in gas barrier coatings). These nanoencapsulates are useful to create nanoemulsions that stabilize hydrosoluble compounds with high oxygen barrier. In a particular embodiment, the hydrosoluble oxygen barrier compounds are polyvinyl alcohol, cationic or anionic polyvinyl alcohol derivates (Mowiol^{™} product range from Kuraray^{®}), Gantrez^{™} polymeric line (from Ashland^{®}), polymethyl vinyl ether-co-maleic acid, Acetylated polyvinyl alcohol or carbohydrates.

In a particular embodiment, the multi-walled water-in-oil-in-water nanoencapsulate as defined above is that wherein the oil droplet further comprises a chemical/biological active compound solubilized in the oil phase.

In another particular embodiment, the multi-walled water-in-oil-in-water nanoencapsulate as defined above is that which encapsulates a hydrophilic drug in the central core and a liposoluble drug of different action (e.g., cytotoxic) in the oil phase. Both active compounds may have different accumulation and release systems according to the corresponding wall compositions.

In another particular embodiment, the multi-walled water-in-oil-in-water nanoencapsulate as defined above is that which the polyurethane-polyurea walls are functionalized in such a way that allows a selective release of each wall, thereby, providing each of the walls with a different release system, for instance by oxidation or reduction, or by hydrolytic enzymes (esterases, lipases, proteases, carbohydrate hydrolases, etc.).

The process for preparing the water-in-oil-in-water multi-walled polymeric nanoencapsulate of the present invention is also part of the invention. The process comprises the following steps:
a) first carrying out a first encapsulation W/O by a process comprising the following steps: a1) Mixing a water-soluble chemical/biological active compound, a prepolymer 1), and a water-soluble polyamine 1, in a given volume of water; a2) Adding between 5 7-fold more amount of an organic solvent immiscible in water to generate a first emulsion by means of a phase inversion; a3) Adding a polyisocyanate 1 soluble in the organic solvent selected for the emulsion to create the wall of the first encapsulation for the water-soluble chemical/biological agent through its stoichiometric reaction at the interface with the amines of the prepolymer 1 and the water-soluble polyamine; b) Carrying out a second encapsulation O/W by a process comprising the following steps: b1) Adding to the previous encapsulation a prepolymer 3 which is the corresponding precursor of prepolymer 2 with secondary amine terminal groups instead of isocyanate groups; b2) Adding the mixture of step b1) over an equivalent excess of polyisocyanate from the equivalent amount of the prepolymer 3 to form a prepolymer 2 *in situ*; b3) Adding the necessary amount of water to generate a second emulsion by means of a second phase inversion; b4) Adding a water-soluble polyamine 2 to create the wall of the second encapsulation through its reaction at the interface with the isocyanate from prepolymer 2 and the free isocyanate added in excess; c) Optionally, evaporating the organic solvents; d) Optionally, dialyzing the water in oil in water emulsion of step c); wherein prepolymer 1, prepolymer 2, and prepolymer 3 have independently a main chain and side chains of different polarities; the main chains of each of the prepolymers comprises in its backbone urethane functional groups, urea functional groups, polyethylene oxide repeating units, disulfide bonds, amine bonds, and optionally hydrophobic repeating units; and two end terminal functional groups; the two-end terminal functional groups of prepolymer 1 are secondary amine terminal groups; and the two end terminal functional groups of prepolymer 2 are isocyanate terminal groups; the two end terminal functional groups of prepolymer 3 are secondary amine terminal groups.

In a particular embodiment, the main chain of any of the prepolymers 1, 2 or 3 comprises hydrophobic repeating units.

The use of prepolymer 3 which is an amine-terminated polymer susceptible to be reactivated *in situ* during the nanoencapsulation step to yield the corresponding NCO-reactive polymer allows using these types of prepolymers containing hydrophilic groups such as PEG which are sensitive to hydrolysis and tend to lose NCO content over time. Prepolymer 3 is soluble in the organic solvent.

As mentioned above, both prepolymers prepolymer 1 and prepolymer 2 are self-emulsifiers: prepolymer 1 has emulsifying capacity in the moment when the water immiscible solvent is at a higher concentration than the water in the reaction vessel. The prepolymer 2 has emulsifying capacity at the moment when water is at a higher concentration than the organic solvent in the reaction vessel. Thus, in another particular embodiment, the multi-walled water-in-oil-in-water nanoencapsulate as defined above is that which is absence of an emulsifier agent. This is advantageous because it avoids the addition and subsequent removal of emulsifier agents once the encapsulates are synthesized.

The reactions can easily be controlled by IR spectroscopy given that NCO has a very clear and characteristic stretching band at 2280-2230 cm⁻¹.

In a particular embodiment, with any of the embodiments above or below, the polyurethane/polyurea composition of the prepolymer 3 have a HLB value higher than the HLB of the polyurethane/polyurea composition of prepolymer 1.

In another particular embodiment, with any of the embodiments above or below, the amine groups of the prepolymer 1 have a HLB value equal to or lower than 10. In another particular embodiment, with any of the embodiments above or below, the amine groups of the prepolymer 3 have a HLB value equal to or higher than 10.

In a particular embodiment in combination with any of the embodiments above or below, the solvent in the step a2) is added slowly with high agitation.

In another particular embodiment, with any of the embodiments above or below, steps a) and b) are carried out at room temperature. In another particular embodiment, with any of the embodiments above or below, step c) is carried out at a low pressure and at a temperature comprised in the range 35- 40°C.

In another particular embodiment, with any of the embodiments above or below, the first encapsulation (step a2) is carried out using a water immiscible solvent which can be (C₆-C₇)alkanes, (C₆-C₉)-aromatic hydrocarbons, or chlorinated solvents (e.g., cyclohexane, hexane, toluene, chloroform, or dichloromethane). In another particular embodiment, with any of the embodiments above or below, the first encapsulation, a certain rotation speed (from 200 rpm to 500 rpm) is used in order to form the emulsion.

In another particular embodiment, with any of the embodiments above or below, capsules have also been synthesized with a high boiling point organic oil as an intermediate organic phase (a W/O/W system in which a liposoluble compound could be charged in the organic phase between the two walls dissolved in this oil).

In another particular embodiment, with any of the embodiments above or below, the dialysis is carried out between 24-72h in water, with a 10kDa membrane that removes molecules that have been left in the last aqueous phase (non-encapsulated drug, a slight excess of unreacted amine, etc.).

The product obtained is a capsule W/O/W that has two synthesized nanostructured walls.

The solvent removal allows to obtain a high stabilized with a hydrodynamic diameter from 20 to 500 nm w/o/w nanoencapsulate (hydrodynamic diameter, by DLS). In a particular embodiment, the hydrodynamic diameter is from 20 to 100 nm. In another particular embodiment, the hydrodynamic diameter is from 20 to 50 nm.

In a particular embodiment, with any of the embodiments above or below, the organic solvents are evaporated at low pressure and moderate temperature (35- 40°C). When the intermediate solvent is extracted to get a hydrophilic core (with the chemical/biological compound dissolved), the wall formed by the Prepolymer 1 is in direct contact with the wall formed by the Prepolymer 2 and water as a dispersant phase (capsules W // W').

Capsules have also been prepared in which, just after first encapsulation, an amino acid is added that reacts with part of the isocyanate groups and introduces into the wall carboxyl groups that define an amphoteric behavior in terms of surface charge (this is the method used to accumulate selectively in cancer cells).

The water-in-oil-in-water multi-walled polymeric nanoencapsulate of the present invention can be defined by its preparation process. Thus, it is part of the invention a water-in-oil-in-water multi-walled polymeric nanoencapsulate obtainable by the process as defined above, including any of the particular and/or preferred embodiments disclosed above.

It is also part of the invention, the multi-walled water-in-oil-in-water nanoencapsulate as defined above is that which encapsulates a hydrophilic drug in the central core and a drug of different action (e.g.: cytotoxic) liposoluble in the oil phase for use in the treatment of cancer.

It is also part of the invention, the use of a multi-walled water-in-oil-in-water nanoencapsulate as defined above which encapsulate water-soluble fluorophores NIR or Far-red in techniques such as Fluorescence-guided Surgery. Examples of such flurophores are DAPI, ICG, and propidium iodide.

Liposomes can load both hydrophilic and hydrophobic drugs, which are entrapped in the aqueous core and in the lipid phase, respectively. They present clear advantages in terms of biocompatibility and wide applicability. It is also part of the invention a multi-walled water-in-oil-in-water liposome. Another aspect of the present invention relates to a hybrid water-in-oil-in-water polyurethane/polyurea liposome comprising internal water droplets which are dispersed within larger oil droplets, wherein: a) the internal water droplets comprise a chemical/biological active compound solubilized in the water and a first polyurethane-polyurea polymeric wall; the first polyurethane-polyurea polymeric wall is obtainable in a water-in-oil emulsification process by phase inversion through reaction at the interface of a polyisocyanate 1 with the amine groups of both (a) a first polyurethane/polyurea polymer (prepolymer 1), and (b) a water-soluble polyamine 1; prepolymer 1 have a main chain and side chains of different polarities; the main chains comprise in its backbone urethane functional groups, urea functional groups, polyethylene oxide repeating units, disulfide bonds, amine bonds, and hydrophobic repeating units; and two end terminal functional groups; the two-end terminal functional groups of prepolymer 1 are secondary amine terminal groups; and b) the oil droplets comprise the internal water droplets dispersed therein and an outer wall which resembles the structure of a liposome; the outer wall is obtainable in an oil-in-water emulsification through surface arrangement of di-palmitoyl-phosphatidylcholine and cholesterol.

In a particular embodiment, with any of the embodiments above or below, the main chain of the prepolymer 1 comprises hydrophobic repeating units.

All the particular embodiments of the multi-walled water-in-oil-in-water nanoencapsulate with respect to the internal water droplets are also particular embodiments of the hybrid water-in-oil-in-water polyurethane/polyurea liposome.

It is also part of the invention a process for preparing an hybrid water-in-oil-in-water polyurethane/polyurea liposome, the process comprises:
a) first carrying out a first encapsulation W/O by a process comprising the following steps: a1) Mixing a water-soluble chemical/biological active compound, a prepolymer 1), and a water-soluble polyamine 1, in a given volume of water (between 0.05 to 0.5 mL); a2) Adding the necessary amount of an organic solvent immiscible in water to generate a first emulsion by means of a phase inversion; a3) Adding a polyisocyanate 1 soluble in the organic solvent selected for the emulsion to create the wall of the first encapsulation for the water-soluble chemical/biological agent through its reaction at the interface with the amines of the prepolymer 1 and the water-soluble polyamine;
   wherein prepolymer 1 have a main chain and side chains of different polarities; the main chains of each of the prepolymer 1 comprises in its backbone urethane functional groups, urea functional groups, polyethylene oxide repeating units, hydrophobic repeating units, disulfide bonds, and amine bonds; and two end terminal functional groups; the two-end terminal functional groups of prepolymer 1 are secondary amine terminal groups;
b) carrying out a second encapsulation O/W by a process comprising the following steps:
   b1) Adding to the previous encapsulation di-palmitoyl-phosphatidylcholine in a first suitable solvent and cholesterol in a second suitable solvent;
   b2) Adding the necessary amount of water to generate a second emulsion by means of a second phase inversion and create an outer wall through superficial arrangement of di-palmitoyl-phosphatidylcholine and cholesterol;
c) Optionally, evaporating the organic solvents;
d) Optionally, dialyzing the water-in-oil-in-water emulsion of step c);

In a particular embodiment, the main chain of the prepolymer 1 comprises hydrophobic repeating units.

An example of a suitable solvent for the di-palmitoyl-phosphatidylcholine is ethanol. An example of a suitable solvent for the cholesterol is tetrahydrofuran.

In a particular embodiment, with any of the embodiments above or below, the cholesterol/DPPC molar ratio is in a range from 1: 4 to1:6. In another particular embodiment the cholesterol/DPPC molar ratio is in a range from 1: 5 to1:6. In another particular embodiment, the molar ratio of cholesterol/DPPC is 1:5.85. When the decrease de cholesterol/DPPC ratio, smaller structures can be obtained. With a cholesterol/DPPC with ratio of about 1:6 lipid wrapped nanoencapsulates about 20-80 nm are obtained.

In a particular embodiment, with any of the embodiments above or below, the organic solvents are removed from the lipid wrapped nanoencapsulate.

In a particular embodiment, with any of the embodiments above or below, the organic solvents are evaporated at low pressure and moderate temperature (30- 40°C). When the intermediate solvent is extracted to get a hydrophilic core (with the chemical / biological compound dissolved), the wall formed by the Prepolymer 1 is in direct contact with the wall formed by the liposome and water as a dispersant phase.

In another particular embodiment, with any of the embodiments above or below, dialyzing the sample is carried out between 24-72h in water, in particular MilliQ water, with a 10kDa membrane that removes molecules that have been left in the last aqueous phase (non-encapsulated drug, a slight excess of unreacted amine, etc.).

The hybrid water-in-oil-in-water polyurethane/polyurea liposome of the present invention can also be defined by its preparation process. Thus, it is part of the invention the hybrid water-in-oil-in-water polyurethane/polyurea liposome obtainable by the process defined above, including any particular embodiment or preferred embodiment of the process.

Another aspect of the present invention relates to a composition comprising a nano encapsulate as defined above or the hybrid water-in-oil-in-water polyurethane/polyurea liposome together with carriers.

In a particular embodiment, with any of the embodiments above or below, biological groups, like gangliosides, can be introduced by hydrophobic interactions in the external liposome-like wall, i.e. it as a carrier. In a particular embodiment, the ganglioside is Galβ1,3GalNAcβ1,4(Neu5Acα2,3)Galβ1,4Glcβ1,1 Ceramide (GM1), in which hybrid targeted nanocapsules are intended to use against HIV-infection.

Another aspect of the present invention relates to a cosmetic or pharmaceutical composition comprising a nano encapsulate as defined above, or the liposome as defined above, together with cosmetically or pharmaceutically acceptable excipients and/or carriers.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

### Example 1: Preparation of an amphiphilic polymer (for the first encapsulation)

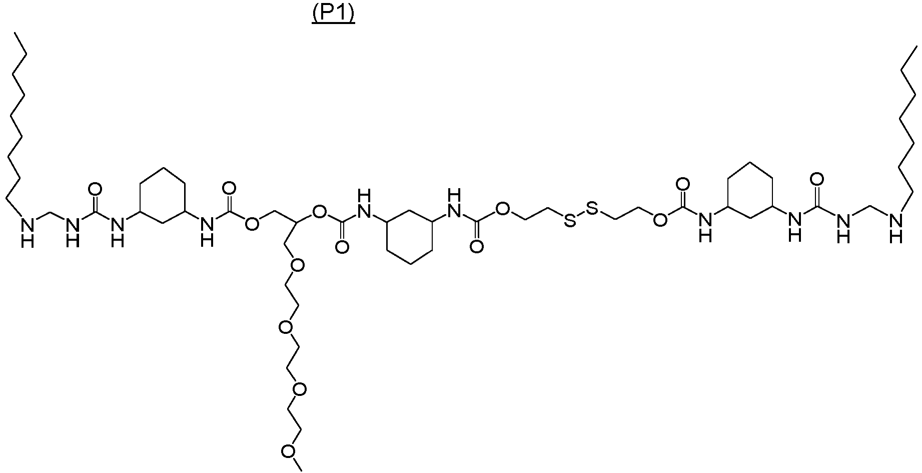

2,2'-Dihydroxyethyl disulfide (DEDS) (2.87 g , 37.25 meq) and diol-polyethylene glycol 1000 (YMER N-120) (3.64 g, 7.02 meq) were added into a three-necked round-bottom flask equipped with mechanical stirring at room temperature (rt) and purged with N2. When the mixture was homogeneous, isophorone diisocyanate (IPDI) (8.89 g, 79.98 meq) was added into the reaction vessel under gentle mechanical stirring and reaction was heated to 60°C. The polyaddition reaction was kept under these conditions until the NCO stretching band intensity did not change, monitored by IR spectroscopy. At this point, dry acetone (17.56 mL) was added into the reaction mixture in order to fluidify the polymer. In parallel, 1,3-diamino-N-octadecylpropane (Genamin TAP 100D) (6.85 g, 41.98 meq) was dissolved with dry THF (5.53 mL) into another 100 mL three-necked round-bottom flask, which had previously been purged with N2. The former reaction mixture was added dropwise onto the latter under half-moon 100 rpm mechanical stirring. The reaction was monitored by IR until the NCO stretching band intensity had completely disappeared.

The polymerization reaction is controlled by FTIR spectroscopy given that NCO has a very clear and characteristic stretching band at 2280-2230 cm⁻¹ (see FIG.2). FIG. 2 also shows a successful polymerization reaction between diols, the diamine and the diisocyanate, in both steps of the polymer synthesis. The IR spectra performed during the first step of the synthesis confirmed polyurethane bond formation along with NCO consumption. The IR spectra performed during the second step of the synthesis also confirmed polyurea formation.

### Example 2: Preparation of an amphiphilic cationic polymer (for the second encapsulation) (P2)

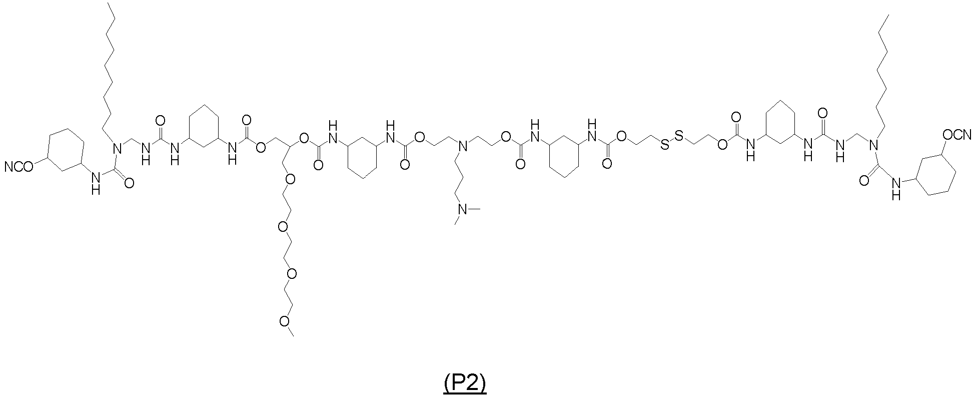

2,2'-Dihydroxyethyl disulfide (DEDS) (901.0 mg, 11.68 meq), diol-polyethylene glycol 1000 (YMER N-120) (12.04 g, 23.18 meq) and N-(3-dimethylaminopropyl)-N,N'-diisopropanolamine (Jeffcat DPA) (981.3 mg, 8.99 meq) were added into a three-necked round-bottom flask equipped with mechanical stirring at room temperature (rt) and purged with N2. When the mixture was homogeneous, isophorone diisocyanate (IPDI) (8.14 g, 73.24 meq) was added into the reaction vessel under gentle mechanical stirring. The polyaddition reaction was kept under these conditions until the NCO stretching band intensity did not change, monitored by IR spectroscopy. At this point, dry THF (21 mL) was added into the reaction mixture in order to fluidify the polymer. In parallel, 1,3-diamino-N-octadecylpropane (Genamin TAP 100D) (5.99 g, 35.45 meq) was dissolved with dry THF (5.23 mL) into another 100 mL three-necked round-bottom flask, which had previously been purged with N2. The former reaction mixture was added dropwise onto the latter under half-moon 100 rpm mechanical stirring. The reaction was monitored by IR until the NCO stretching band intensity had completely disappeared.

The polymerization reaction is controlled by FTIR spectroscopy given that NCO has a very clear and characteristic stretching band at 2280-2230 cm⁻¹ (see FIG.1). FIG. 1 shows a successful polymerization reaction between diols, the diamine and the diisocyanate, in both steps of the polymer synthesis. The IR spectra performed during the first step of the synthesis confirmed polyurethane bond formation along with NCO consumption. The IR spectra performed during the second step of the synthesis also confirmed polyurea formation.

### Example 3: Preparation of a water in oil (W/O) encapsulate

Hydrophilic active compound (X mg, Y µmol), MilliQ water (100 mg), polymer P1 (635.2mg, 0.089 meq), DETA 10% w/w (45.0 mg, 0.131 meq) and dry acetone (1 mL) were mixed in a vial with magnetic stirring and homogenized, with ultrasonication if it is necessary, for 15 min protected from light. Cyclohexane (1mL) was added drop wise to the vial to perform first inverse phase emulsion. Then, IPDI (40.0 mg, 0.36 meq) was added to crosslink the first nanocapsules wall and the NCO presence is controlled by FTIR. This synthetic process furnishes NCs1. The O/W encapsulation reaction was controlled by FTIR spectroscopy in order to certify the slight presence of free NCO at the end of the encapsulation, which reacts on the first step of the second encapsulation (see FIG .3).

### Example 4: Preparation of a water in oil in water (W/O/W) encapsulate (encapsulation of the NCs1)

IPDI (225.0 mg, 2.02 meq) was added into a three-necked round-bottom flask equipped with mechanical stirring purged with N2 and protected from light. In parallel, NCs1 (1.50 g), polymer P2(5.87 g, 0.62 meq) and dry THF (1 mL) were mixed in a vial, added into the flask and homogenized for 10 min at 150 rpm, protected from light. At this point, an alkaline aqueous solution of L-lysine was prepared by dissolving 0.93 g L-lysine in 11.38 g of Milli-Q water and adjusting pH to 11.0 with alkaline NaOH solutions at 3 M and 1 M (total L-lysine concentration 7.56% by wt). This L-lysine solution (851.1 mg, 0.78 meq) was added at 450 rpm and the polyaddition reaction was checked after 1 min by FTIR. Then, the organic phase was emulsified at 450 rpm with cold Milli-Q water (12.0 g) and finally a 10% w/w aqueous solution of diethylenetriamine (DETA) (253.3 mg of DETA, 0.74 meq) was added in order to generate crosslinked NCs from the nano micelles. The stirring was reduced to 150 rpm. This polyaddition reaction was monitored by IR and pH measurements. Once the NCs were formed, THF was removed from the reactor at 35 °C under vacuum and the dialysis purification, against Milli-Q water for 24 h using a Spectra/Por molecular porous membrane tubing with a 12-14 kDa molecular MWCO, was carried out. This synthetic procedure furnishes NCs2.

**Table 1. Amounts of reagents used to prepare different NCs2:**

| Substance | X (mg) | Y (µmol) |
|---|---|---|
| H₂O (reference) | - | - |
| ICG | 10.00 | 12.90 |
| Resiquimod (R848) | 25.00 | 79.52 |
| DAPI | 5.00 | 18.03 |
| Allophycocyanin | 1.00 | 0.001 |
| Cisplatin | 27.00 | 90.43 |
| Propidium Iodide | 5.00 | 7.48 |

The W/O/W encapsulation reaction was also controlled by FTIR spectroscopy as shown FIG. 4. IR spectra of the NCs (regardless their loading) indicated also a successful nanocapsule formation. The purple line in the IR spectra represents the sample after the P1+NCs1 mixing. The yellow line corresponds to the P1+NCs1 mixture added over determinate diisocyanate amount. This initial step was the reactivation of the polymer and its conversion to an NCO-reactive entity. Afterwards, L-lysine sodium salt was added (green line) and reacted with the activated polymer. A decrease on the intensity of the NCO stretching band at 2255 cm⁻¹, concomitantly with an increase of the carbonyl and CN stretching bands, confirmed urea formation (1642 cm⁻¹ and 1532 cm⁻¹, respectively). In blue line, NCO stretching band is maintained where is represented the phase inversion with MilliQ water. Finally, the triamine was added (red line) and the NCO stretching band instantaneously disappeared and the urea-associated bands increased their intensity as a result of the rapid reaction between remaining NCO groups and this polyamine.

### Example 5: Preparation of a water in oil in water (W/O/W) lipidic dispersion (encapsulation of the NCs1 using amphiphilic lipids. External liposome structure)

First, a water in oil (W/O) encapsulate as disclosed in Example 3 was prepared (NCs1).

Then, NCs1 (1.00 g), was added into a three-necked round-bottom flask equipped with mechanical stirring purged with N2 and protected from light. In parallel, one solution containing Di-palmitoyl-phosphatidylcholine (DPPC, 42.3 mg) in ethanol (2.78 mL) and another solution of cholesterol (16.00 mg) in THF (0.62 mL), were prepared, added into the flask and homogenized for 5 min at 150 rpm, protected from light. Then, the organic phase was dropwise emulsified at 400 rpm with cold Milli-Q water (10.0 g) Once the lipid-wrapped NCs were formed, Ciclohexane, THF and EtOH were removed from the reactor at 35 °C under vacuum and the dialysis purification, against Milli-Q water for 24 h using a Spectra/Por molecular porous membrane tubing with a 12-14 kDa molecular MWCO, was carried out. This synthetic procedure furnishes NCs2.

This synthetic procedure reaches the formation of lipid wrapped NCs2 about 300-700 nm (hydrodynamic diameter, by DLS).If cholesterol/DPPC ratio is decreased, using the amounts detailed in table 2, smaller structures are obtained, about 20-300 nm.

**Table 2:**

| W/O/W lipid wrapped NCs2 | Amount | Units of measurement |
|---|---|---|
| NCs1 | 1,0025 | g |
| DPPC | 0,0398 | g |
| Cholesterol | 0,0068 | g |
| Ethanol | 2,7778 | mL |
| THF | 0,5910 | mL |
| H2O | 10,0000 | g |

### Example 6: Physicochemical characterization of NCs1 with carboplatin and carboplatin content determination

### Nanocapsules tested:

- JB-H2O-PPAH-PP22-Lys-02. Amphoteric NCs loaded with water (control sample). Dialyzed in MilliQ water. Water-loaded NCs (reference) prepared following example 3.
- JB-carboPt-PPAH-PP22-Lys-01. Amphoteric NCs loaded with carboplatin in water media. Dialyzed in MilliQ water. Carboplatin-loaded NCs prepared following example 3.

Physicochemical characterization: The NPs were characterized by different analytical techniques. Firstly, the synthetic process was monitored by Infrared Spectroscopy (IR) and pH measurements to confirm that the final polyamine crosslinked the remaining reactive sites along the NP shell. Afterwards, the NPs were purified by dialysis against Milli-Q water for 24 h and the resulting product was characterized by Dynamic Light Scattering (DLS) to determine the average particle size in a solid content concentration between 0.05 to 0.1%, by ζ -potential (ζ -pot) to analyze the surface charge in different pH conditions and by ICP-MS to determine the carboplatin payload. All the samples were assayed in triplicate in each technique.

Size: The average size for each sample is shown in Table 3:

| Sample | Size ± SD (nm) |
|---|---|
| JB-H2O-PPAH-PP22-Lys-02 | 19.19 ± 3.12 |
| JB-carboPt-PPAH-PP22-Lys-01 | 19.74 ± 1.61 |

Z-potential: the ζ-potential values (surface charge) of the NPs are shown in Table 4:

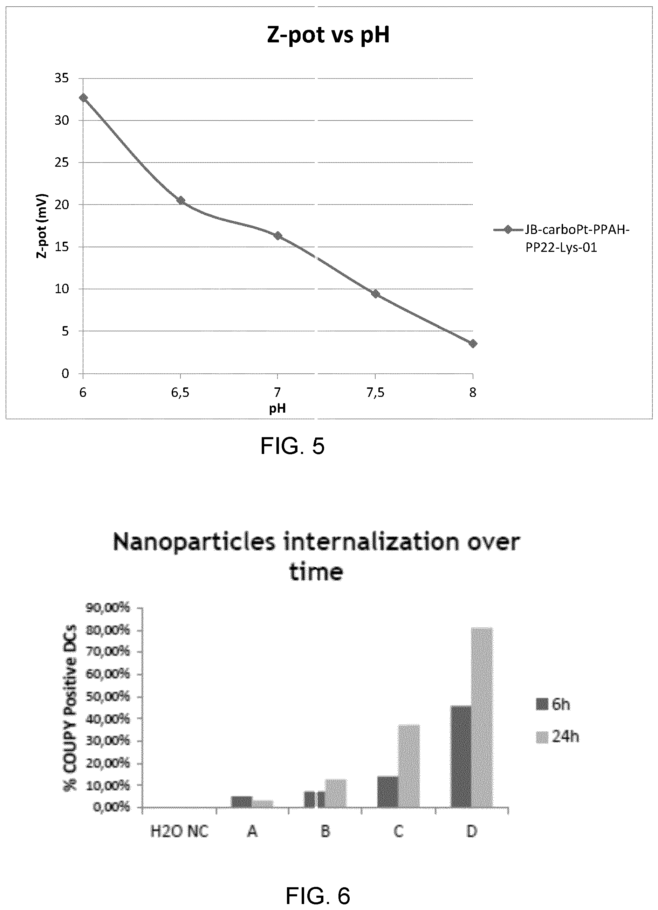

FIG. 5 shows that drug loaded amphoteric NCs can be prepared using the described

| Sample | ζ-Pot ± SD (mV) at pH=6.0 | ζ -Pot ± SD (mV) at pH=6.5 | ζ -Pot ± SD (mV) at pH=7.0 | ζ -Pot ± SD (mV) at pH=7.5 | ζ -Pot ± SD (mV) at pH=8.0 |
|---|---|---|---|---|---|
| JB-H2O-PPAH-PP22-Lys-02 | - | - | - | - | - |
| JB-carboPt-PPAH-PP22-Lys-01 | 32.7 ± 0.3 | 20.5 ± 0.35 | 16.3 ± 0.4 | 9.43 ± 0.35 | 3.51 ± 0.13 |

method.

NCs and carboplatin content: The concentration of NCs in the emulsions was quantified with a solids concentrator that is commonly used in polymer chemistry. In Table 5, the concentration of NCs in the final emulsion is expressed in mg/mL Moreover, the concentration of carboplatin inside the NCs was determined by ICP-MS analysis following the method described below.

**Table 5: Concentration of NCs and carboplatin in each sample.**

| Sample | [NCs] (mg/mL) | [carboplatin] (mg/mL) | [carboplatin] (µM) |
|---|---|---|---|
| JB-H2O-PPAH-PP22-Lys-02 | 140.68±0.05 | - | - |
| JB-carboPt-PPAH-PP22-Lys-01 | 147.83±0.33 | 0.0023 ± 0.0004 | 6.17 ± 1.04 |

20 µL of emulsion samples were diluted in 500 µL of concentrated 72% v/v nitric acid, and the samples were then transferred into wheaton v-vials (Sigma-Aldrich) and heated in an oven at 373 K for 18 h. The vials were then allowed to cool, and each sample solution was transferred into a volumetric tube and combined with washings with Milli-Q water (1.5 mL).

Digested samples were diluted 5 times with Milli-Q to obtain a final HNO3 concentration of approximately 3.6% v/v. Platinum content was analyzed on an ICP-MS Perkin Elmer Elan 6000 series instrument at the Centres Cientifics i Tecnològics of the Universitat de Barcelona. The solvent used for all ICP-MS experiments was Milli-Q water with 1% HNO₃. The platinum standard (High-Purity Standards, 1000 µg/mL + 5 µg/mL in 5% HNO₃) was diluted with 1% HNO₃ to 20 ppb. Platinum standards were freshly prepared in Milli-Q water with 1% HNO₃ before each experiment. The concentrations used for the calibration curve were in all cases 0, 0.2, 0.4, 1, and 2 ppb. The isotope detected was 196Pt and readings were made in triplicate. Rhodium was added as an internal standard at a concentration of 10 ppb in all samples.

### Example 7: Physicochemical characterization of NCS DAPI and ICG-1 and fluorophore content determination

### The nanocapsules (NCs) tested:

- NI-H₂O-PPAH-PP22-LYS-03. NCs without any fluorophore, (control sample).
- NI-DAPI-PPAH-PP22-LYS-01. NCs loaded with fluorophore DAPI.λ_{exc:} 358 nm / λ_{em:} 461 nm 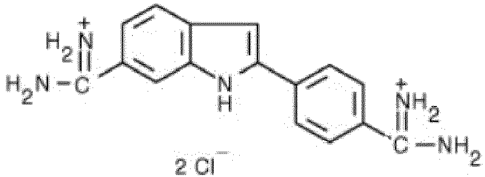
- NI-ICG-PPAH-PP22-LYS-01. NCs loaded with fluorophore Indocyanine Green (ICG): λ_{exc:} 789 nm / λ_{em:} 814 nm.

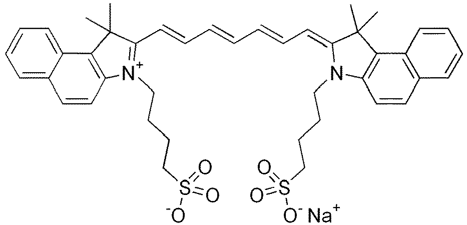

Physicochemical characterization: The NCs were characterized by different analytical techniques. Firstly, the synthetic process was monitored by Infrared Spectroscopy (IR) and pH measurements to confirm that the final polyamine crosslinks the remaining reactive sites along the nanoparticle (NP) shell. Afterwards, the NPs were purified by dialysis against Milli-Q water for 24 h and the resulting product was characterized by Dynamic Light Scattering (DLS) (solid content concentration between 0.05 to 0.1%) to determine the average particle size, by ζ-potential (ζ-pot) (solid content concentration between 0.05 to 0.1%) to analyze the surface charge in different pH conditions and by UV/Vis spectroscopy (calibration curve performed in water in a range of Absorbance between 0 to 1 A.U., where it is directly proportional to concentration) to determine the DAPI and ICG payload. All the samples were assayed in triplicate in each technique.

Size: The average sizes of samples are shown in Table 6:

| Sample | Size ± SD (nm) |
|---|---|
| NI-H₂O-PPAH-PP22-LYS-03 | 20.3 ± 2.2 |
| NI-DAPI-PPAH-PP22-LYS-01 | 30.2 ± 9.3 |
| NI-ICG-PPAH-PP22-LYS-01 | 13.2 ± 2.4 |

Z-potential: Also, the ζ-potential values (surface charge) of the NCs are studied to elucidate if these are amphoteric and depending on the pH of the medium, the surface charge suffers any change. Results are shown in :

**Table 7**

| Table 7 C-potential values of the samples | | | | | |
|---|---|---|---|---|---|
| Sample | ζ-Pot ± SD (mV) at pH=6.0 | ζ-Pot ± SD (mV) at pH=6.5 | ζ-Pot ± SD (mV) at pH=7.0 | ζ-Pot ± SD (mV) at pH=7.5 | ζ-Pot ± SD (mV) at pH=8.0 |
| NI-H₂O-PPAH-PP22-LYS-03 | 33.5 ± 0.2 | 28.5 ± 0.5 | 21.3 ± 0.8 | 15.5 ± 0.1 | 11.6 ± 0.6 |
| NI-DAPI-PPAH-PP22-LYS-01 | 34.8 ± 0.2 | 27.8 ± 0.7 | 23.1 ± 0.2 | 9.9 ± 0.3 | -4.1 ±0.2 |
| NI-ICG-PPAH-PP22-LYS-01 | 36.4 ± 0.9 | 31.1 ± 0.3 | 26.3 ± 1.4 | 13.9 ± 0.4 | 7.4 ± 0.6 |

Table 7, it can be observed a different surface charge depending on the pH of the medium. As increasing the pH of the medium from 6.0 to 8.0, the ζ-potential values decrease in all three samples and in the case of the NCs loaded with DAPI, this value change to negative.

Fluorophores and NCs content: The concentration of the ICG inside the NCs was determined by UV/Vis spectroscopy after performing a calibration curve with the free fluorophore. 0.25 mL of ICG NCs emulsion samples were diluted in 25 mL volumetric flask with Milli-Q water. The diluted samples (for triplicate) were analyzed by UV-Vis in the range of 650 nm to 950 nm. The ICG peak was evaluated at 798nm. In the case of NCs loaded with DAPI, only a qualitative result can be given because it has not been possible to make a calibration curve as for ICG fluorophore. Analysing these samples by UV-Vis and exciting the sample at a range of 450 nm to 200 nm, it can be observed that the fluorophore has been encapsulated and that the maximum absorption of the fluorophore was at 368 nm. The concentration of DAPI NCs in this UV-Vis lecture was 1.58 mg/mL. Also, the concentration of NCs in the emulsions was quantified with a solids concentrator that is commonly used in polymer chemistry. In Table 8, the concentration of NCs in the final emulsion is expressed in mg/mL.

**Table 8: Concentration of NC, DAPI and ICG in each sample**

| Sample | [NCs] (mg/mL) | [DAPI] (mg/mL) | [ICG] (mg/mL) |
|---|---|---|---|
| *NI-H₂O-PPAH-PP22-LYS-03* | 139.5 ± 1.2 | - | - |
| *NI-DAPI-PPAH-PP22-LYS-01* | 158.8 ± 1.0 | *qualitative result | - |
| *NI-ICG-PPAH-PP22-LYS-01* | 143.2 ± 2.0 | - | 0.14 ± 0.003 |

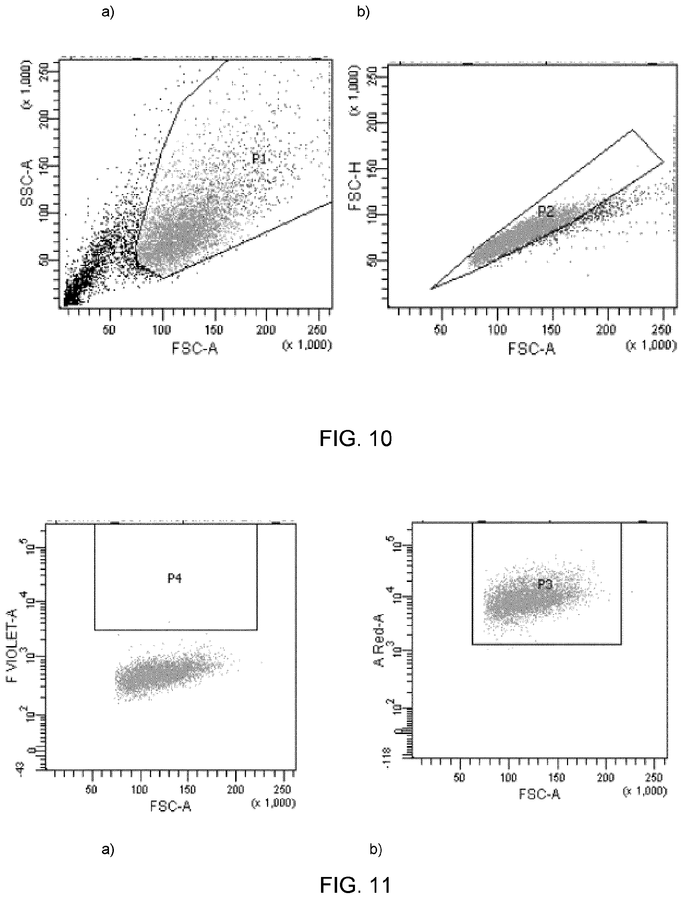

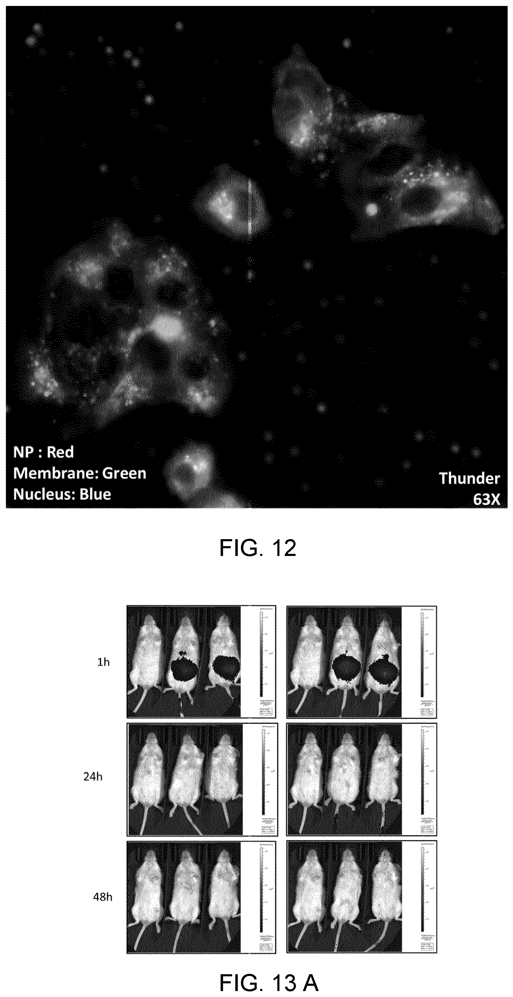

Internalization assays: A flow cytometry analysis, Figures 10 and 11, and Fluorescence Microscopy analysis, Figure 12, for ICG-loaded W/O/W nanocapsules have been performed. Internalization of ICG-loaded W/O/W nanocapsules have been performed in non-differentiated cells at 37°C and 5% CO₂ over 1h. A group of cells that are healthy and well-shaped was selected A fluorescence comparison between non-loaded nanocapsules (reference samples), to avoid autofluorescence phenomena, and ICG-loaded nanocapsules was carried out. It can clearly be observed, in the left plot of Figure 11 (Fig.11a), an absence of fluorescence signal detection in this cell group (non-loaded reference sample). Alternatively, it is observed that almost all the cells incubated with ICG-loaded nanocapsules (Fig.11b) emit fluorescence in the detection zone. Supporting these results, the fluorescence microscopy analysis, shown in Figure 12, detect intracellular signaling for ICG-loaded nanocapsules in non-differentiated hepatic cells.

### Example 8: Effect of Nanocapsules (NCs) loaded with either the TLR7/8 agonist Resiquimod (R848) on dendritic cells

Nanocapsules (NCs) loaded with either the TLR7/8 agonist Resiquimod (R848) (ref. JB-R848-PPAH-PP22-05) or the COUPYSO3 fluorochrome in a MilliQ water core emulsified in MilliQ water were added in vitro to cultured human monocyte-derived dendritic cells (DCs) from healthy subjects to assess the cells immunologic response, as well as the cytotoxicity associated to the NCs.

This was monitored by measuring the expression of co-stimulatory markers on DCs and their viability via flow cytometry (Attune Nxt).

Methodology: DCs were obtained from healthy donor's blood following the procedure on IT-S-IMM-465.

Dosage response assay. Day 7 immature DCs (106 DCs/mL) will be exposed to different concentrations of R848 NCs for 48h in order to assess R848 dose-dependent response and the associated cytotoxicity. Concentrations of nanoencapsulated R848 will be calculated from the experimental R848 value (0.9252 ±0.0699 mg/mL).
- Empty NPs (Control -) (2,16 µL/mL JB-H2O-PPAH-PP22-25)
- 4 µg/mL soluble R848 + MC (10 ng/mL IL-1β, 10 ng/mL IL-6, 10 ng/mL TNFα, 10 mg/mL PGE2) (Control +)
- 0,4 µg/mL R848 (0,43 µL/mL JB-R848-PPAH-PP22-05)
- 1 µg/mL R848 (1,08 µL/mL JB-R848-PPAH-PP22-05)
- 2 µg/mL R848 (2,16 µL/mL JB-R848-PPAH-PP22-05)
- 2,5 µg/mL R848 (2,70 µL/mL JB-R848-PPAH-PP22-05)
- 3 µg/mL R848 (3,24 µL/mL JB-R848-PPAH-PP22-05)
- 3,5 µg/mL R848 (3,78 µL/mL JB-R848-PPAH-PP22-05)
- 4 µg/mL R848 (4,32 µL/mL JB-R848-PPAH-PP22-05)

Each condition will be stained with FITC-labelled αCD80, PE-labelled αCD83 and PE-labelled αIgG as a background fluorescence control and analyzed via flow cytometry.

Additionally, to assess nanoparticle-associated cytotoxicity 3 conditions will be added to the experiment:
- 5 µg/mL R848 (5,40 µL/mL JB-R848-PPAH-PP22-05)
- 6 µg/mL R848 (6,48 µL/mL JB-R848-PPAH-PP22-05)
- 7 µg/mL R848 (7,56 µL/mL JB-R848-PPAH-PP22-05)

All conditions in this assay will be stained with a PerCp5.5-labelled α7-AAD antibody.

Internalization assay. Day 7 immature DCs (106 DCs/mL) were exposed to different concentrations of COUPY NCs for 6h and 24h at 37° or 4° in order to assess NC internalization. Low temperatures impede DCs internalization. Concentrations of nanoencapsulated COUPY were calculated from the experimental polymer value (174.35 ±0.26 mg/mL) in equivalence to the concentrations of polymer used in the dosage-response assay (initial polymer concentration of JB-R848-PPAH-PP22-05 = 249.96 ± 0.50).
- Empty NPs (Control -): 3,29 µL/mL JB-H2O-PPAH-PP22-25
- A: 0,619 µL/mL JB-COUPYSO3-PPAH-PP22-03
- B: 1,55 µL/mL JB-COUPYSO3-PPAH-PP22-03
- C: 3,1 µL/mL JB-COUPYSO3-PPAH-PP22-03
- D: 6,19 µL/mL JB-COUPYSO3-PPAH-PP22-03

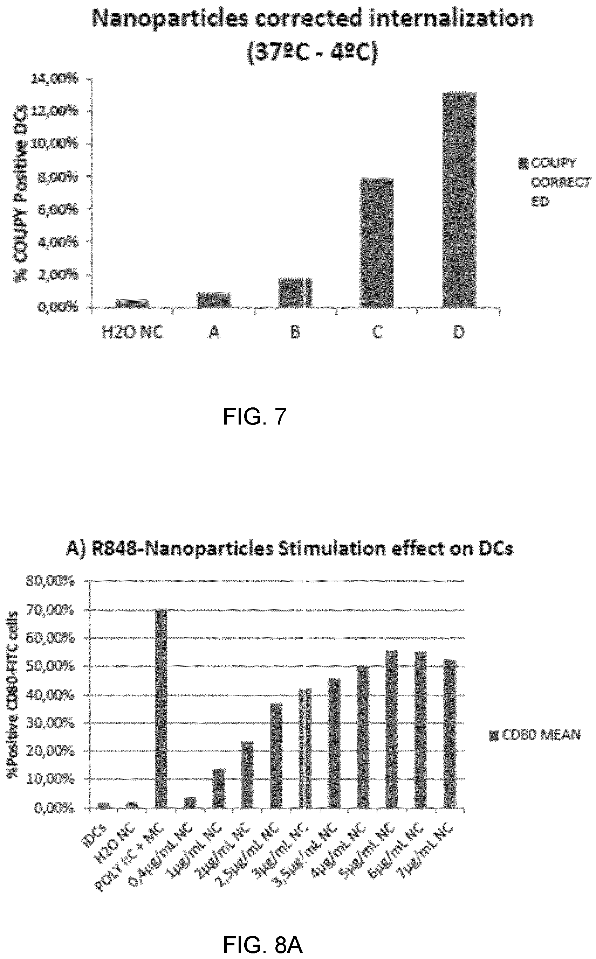

Cells were analyzed by flow cytometry after 6 and 24h incubations. The COUPY fluorochrome is read in the R-PE channel (Green laser, GL-1 channel, Attune NXT cytometer).The results are shown in FIG 6 and FIG. 7. FIG. 6 shows a graphic representation of COUPY expression on DCs, with a negative control where DCs were exposed to JB-H2O-PPAH-PP22-25. The results show that the nanoparticles are internalized and not only bound to their membranes. There's a significant increase in nanoparticles internalization on DCs incubated for 24h with C and D concentrations, in comparison to those with 6h incubations. FIG. 7 shows how the corrected results maintain the internalization ratio of the different NCs concentrations.

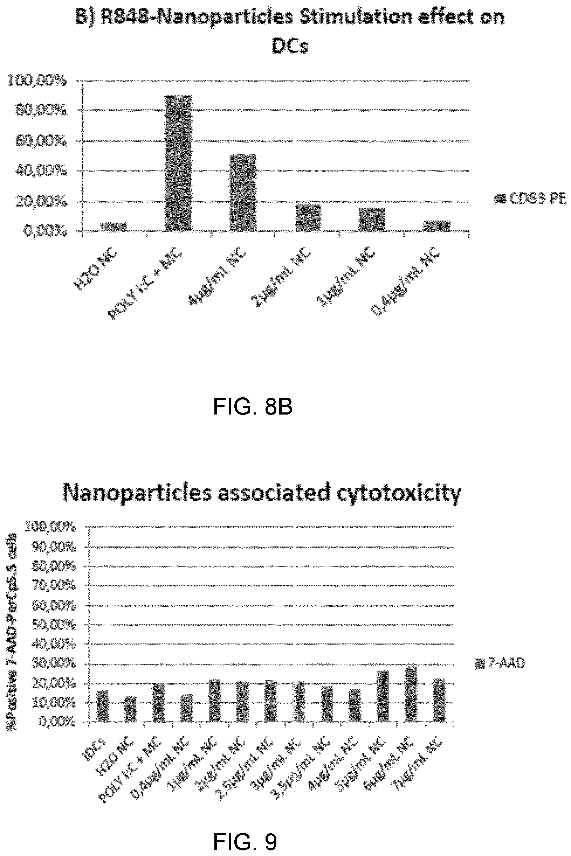

Dose-response assay. FIG. 8 shows graphic representations of CD80 and CD83 expression on DCs, immunogenic maturation markers, with two negative controls - one consisting of immature DCs (iDCs) and the other where DCs were exposed to JB-H2O-PPAH-PP22-25. A combination of Poly I:C and a maturation cocktail of pro-inflammatory cytokines was used as a positive control due to lack of soluble R848 at the time. The results show an exponential increase in CD80 and CD83 expression on DCs at different R848-NP concentrations, peaking at 5 µg R848 per mL of culture.

Cytotoxicity assay. Cytotoxicity was assessed with the viability marker 7-AAD, which stains exposed DNA - in this case from cellular death. Two negative controls were used in the experiment - one consisting of immature DCs (iDCs) and the other where DCs were exposed to JB-H2O-PPAH-PP22-25. Results in a graphic format (FIG. 9) show that there's no significant difference on the associated cytotoxicity of R848-NPs among 0.4 to 4 µg R848 concentrations, whereas it slightly increases with higher concentrations. However, the associated cellular death is inferior to 30% in all cases.

### Example 9: Preparation of a peptide-targeted water in oil in water (W/O/W) encapsulate (encapsulation of the NCs1)

IPDI (225.0 mg, 2.02 meq) is added into a three-necked round-bottom flask equipped with mechanical stirring purged with N2 and protected from light. In parallel, NCs1 (1.50 g), polymer P2(5.87 g, 0.62 meq) and dry THF (1 mL) is mixed in a vial, added into the flask and homogenized for 10 min at 150 rpm, protected from light. Parallelly, a two-end NCO polymer (SP1) is prepared reacting 1.3030 g (1.28 meq) of O,O-Bis(2-aminopropyl) polypropylene glycol-block-polyethylene glycol-block-polypropylene glycol with 0.5711 g (5.14 meq) of IPDI. When NCO-bond disappearance of IR spectra reaches a plateau, 0.25 g of this polymer SP1 is added over a solution of 42.4 mg of cRGDfK in 1 mL of Milli Q water and vigorous sheared using magnetic stirring. At this point, this solution is added at 450 rpm to the three-necked round-bottom flask dropwise. Immediately, the organic phase is emulsified at 450 rpm with cold Milli-Q water (15.0 g) and finally a 10% w/w aqueous solution of diethylenetriamine (DETA) (1.58 g of DETA, 4.6 meq) is added in order to generate crosslinked NCs from the nano micelles. The stirring is reduced to 150 rpm. This polyaddition reaction is monitored by IR and pH measurements. Once the NCs is formed, THF is removed from the reactor at 35 °C under vacuum and the dialysis purification, against Milli-Q water for 24 h using a Spectra/Por molecular porous membrane tubing with a 12-14 kDa molecular MWCO, is carried out. This synthetic procedure furnishes peptide-NCs2.

### Example 10: Preparation of an antibody-targeted water in oil in water (W/O/W) encapsulate (encapsulation of the NCs1)

IPDI (225.0 mg, 2.02 meq) is added into a three-necked round-bottom flask equipped with mechanical stirring purged with N2 and protected from light. In parallel, NCs1 (1.50 g), polymer P2(5.87 g, 0.62 meq) and dry THF (1 mL) is mixed in a vial, added into the flask and homogenized for 10 min at 150 rpm, protected from light. At this point, a commercial solution of 100 µg of anti-GD2 monoclonal antibody in 1 mL of PBS is added at 450 rpm to the three-necked round-bottom flask dropwise. Immediately, the emulsification process, adding 15.0 g of cold Milli-Q water at 450 rpm, takes place and finally a 10% w/w aqueous solution of diethylenetriamine (DETA) (1.58 g of DETA, 4.6 meq) is added in order to generate crosslinked NCs from the nano micelles. The stirring is reduced to 150 rpm. This polyaddition reaction was monitored by IR and pH measurements. Once the NCs is formed, THF is removed from the reactor at 35 °C under vacuum and the dialysis purification, against Milli-Q water for 24 h using a Spectra/Por molecular porous membrane tubing with a 12-14 kDa molecular MWCO, is carried out. This synthetic procedure furnishes mAB-NCs2.

### Example 11: Intravenous administration biodistribution of W/O/W nanocapsules in healthy BALB/C mice model

Intravenous administration biodistribution comparison between ICG, amphoteric ICG-NCs (Example 4 Table 1 for the substance ICG) and cationic ICG-NCs at 1h, 24h and 48h in healthy BALB/C mice model.

Cationic ICG-NCS have been prepared by the same procedure disclosed in Example 4 for the amphoteric ICG-NCs but without adding the solution of L-lysine.

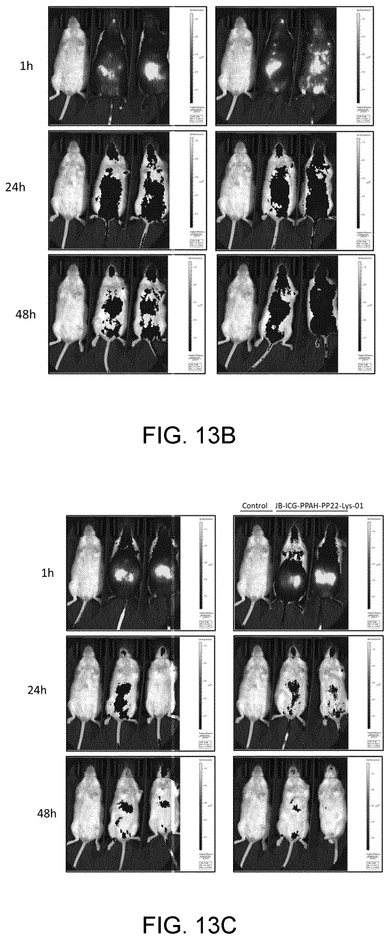

The results can be seen in FIGs 13A-13C.

From the results obtained of intravenous administration and monitoring of both cationic and amphoteric nanocapsules, it can be concluded that they increase the circulation time of ICG in bloodstream.

Cationic ICG-NCs can be trackable for more than 48h after intravenous injection in BALB/C mice model, using IVIS^{®} imaging technology.

### Example 12: In vivo fluorescence imaging biodistribution of W/O/W amphoteric nanocapsules in subcutaneous tumor mice models

A model of immunodepressed mice have been used to conduct this experiment._Female NSG mice (9 weeks old, Charles River) were challenged by subcutaneous (s.c). injection of A375 Human Melanoma cell line (1×10⁶) cells on the right flank with a 23-gauge needle. Amphoteric ICG-NCs and cationic ICG-NCs were administered i.v. at day 14 post-tumor challenge. PBS and ICG free was injected to control groups. Fluorescence was monitoring by in vivo imaging system (IVIS; Perkin Elmer, Waltham, MA, USA,) using excitation/emission wavelength of 780 nm/845 nm.

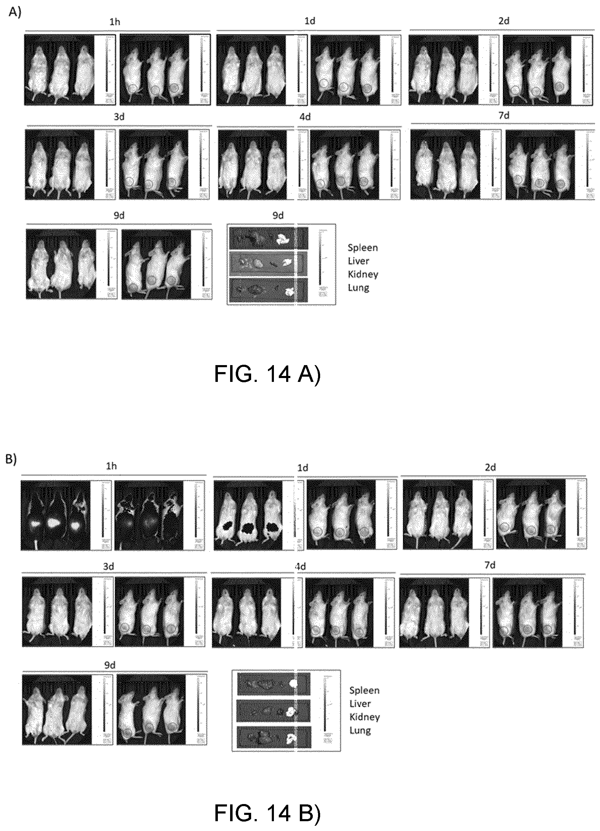

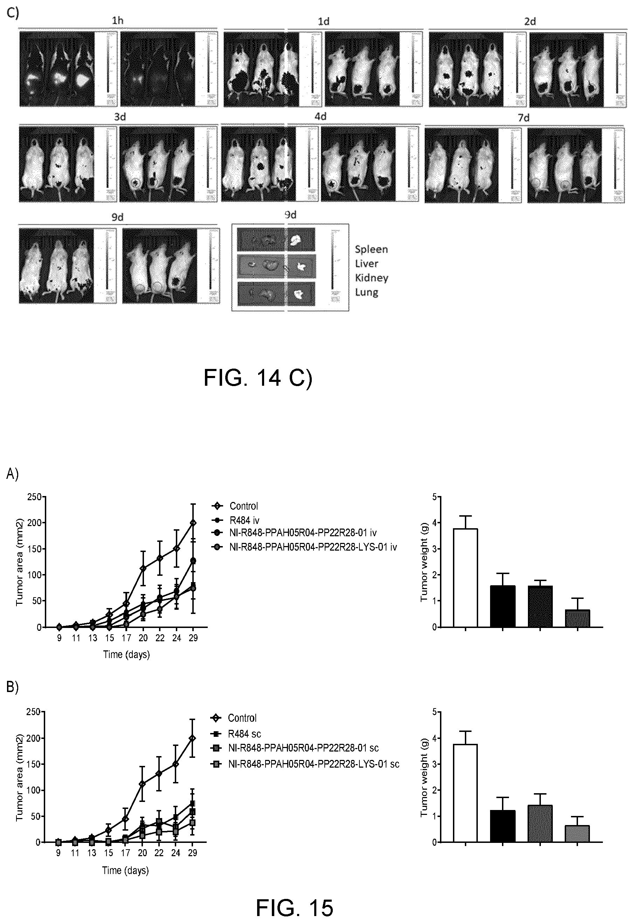

The results are shown in FIG 14. No fluorescence signal is observed in control mice (PBS injection) (see FIG. 14 A). The signal is low when the free ICG fluorofor is injected (see FIG. 14B). Conversely, in NSG tumor (subcutaneous A375) mice model intravenous NCs administration, it is clearly observed specific accumulation for amphoteric ICG-NCs (as disclosed in Example 4 table 1 with ICG as substance), in tumor locations from 3 to 9-days after NCs injection, amphoteric NCs cannot be detected in excreted organs after 9-days of intravenous injection (see FIG: 14C).

### Example 13: Safety for imaging

### (1) Cationic and amphoteric nanocapsules

Encapsulated non-loaded NCS nanocapsules (cationic and amphoteric nanocapsules) were i.v. administered to female BALB/C mice (8 weeks old; Charles River) at three different doses. Blood and serum samples were obtained at 24 h post-administration for acute toxicity analysis (SBCV and SHCV, Universidad Autónoma de Barcelona).

Haematological parameters analysed in blood included white blood cells (WBC) count, red blood cells (RBC) count, haemoglobin (HGB), red blood cell specific volume (HCT), mean corpuscular volume (MCV), mean corpuscular haemoglobin (MCH), mean corpuscular haemoglobin concentration (MCHC), platelet (PLT), lymphocytes (LYMPH), monocytes (MONO), neutrophils (NEU), eosinophils (EO), and basophils (BASO). No significant differences were observed with respect to untreated groups.

Biochemical parameters detected in serum included albumin, alanine aminotransferase (ALT), aspartate aminotransferase (AST), total bilirubin levels (TBIL), creatinine (CREA), gamma-glutamyl transferase (GGT), potassium, total proteins, sodium, and urea. No significant differences were observed with respect to untreated groups
Both cationic and amphoteric NCs did not show associated toxicities in the biochemical and hematological comparison with control healthy group after 24h of intravenous injection.

### (2) Safety for treatment (drug loaded NCs vs free drug)

Encapsulated or free R848 was i.v. administered to female BALB/C mice (8 weeks old; Charles River) at three different doses. Blood and serum samples were obtained at 24 h post-administration for acute toxicity analysis (SBCV and SHCV, Universidad Autónoma de Barcelona).

Haematological parameters analysed in blood included white blood cells (WBC) count, red blood cells (RBC) count, haemoglobin (HGB), red blood cell specific volume (HCT), mean corpuscular volume (MCV), mean corpuscular haemoglobin (MCH), mean corpuscular haemoglobin concentration (MCHC), platelet (PLT), lymphocytes (LYMPH), monocytes (MONO), neutrophils (NEU), eosinophils (EO), and basophils (BASO). No significant differences were observed with respect to untreated groups.

Biochemical parameters detected in serum included albumin, alanine aminotransferase (ALT), aspartate aminotransferase (AST), total bilirubin levels (TBIL), creatinine (CREA), gamma-glutamyl transferase (GGT), potassium, total proteins, sodium, and urea. No significant differences were observed with respect to untreated groups.

### Example 14: Effect of encapsulated R848 administration in melanoma-challenged mice.

R848 is drug that activates immune system. Thus, immune-enabled mice are used in this example.

Female C57BL/6 mice (8 weeks old, Charles River) were challenged by s.c. injection of B16-F0 cells (7×104) on the right flank with a 23-gauge needle. Tumors were measured every other day with a Vernier calliper, and the area (length by width of the tumors, mm2) averaged. R848 amphoteric or cationic containing nanocapsules were administered s.c. or i.v. at day 10 post-tumour challenge. Mice were euthanized at the end of the experiment and tumour weight was measured. The good results in efficacy are shown in FIG. 15.

Taking these results into account it is concluded that s.c. administration of amphoteric R848-NCs seems to promote lower tumor growth together with a higher cytotoxic response against melanoma cells.

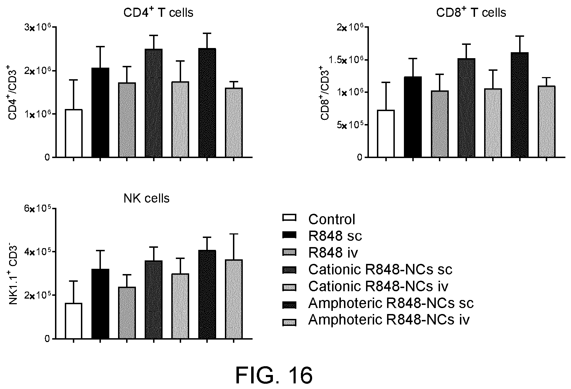

Spleens from the same mice as in FIG. 15 were obtained at the end of the experiment for flow cytometry analysis (FIG. 16). In all cases nanocapsules generate the same or better response than the free drug.

### Citation List

### Patent Literature

- WO0178888 A1
- US4534783A
- US 2004/0115254 A1
- EP2992953A2

### Non Patent Literature

- C. Cusco et al., "Multisensitive drug-loaded polyurethane/polyurea nanocapsules with pH-synchronize shell cationization and redox-triggered release", Polymer Chemistry 2016, vol. 7, pp. 6457-6466).
- Davies et al., "A quantitative kinetic theory of emulsion type, I. Physical chemistry of the emulsifying agent", Gas/Liquid and Liquid/Liquid Interface, Proceedings of the International Congress of Surface Activity, pp. 426-38

## Claims

1. A multi-walled water-in-oil-in-water nanoencapsulate comprising internal water droplets which are dispersed within oil droplets, which are themselves dispersed in an external aqueous continuous phase;
wherein:
the internal water droplets comprise a chemical/biological active compound solubilized in the water and a first polyurethane-polyurea polymeric wall;
the first polyurethane-polyurea polymeric wall is obtainable in a water-in-oil emulsification process by phase inversion through reaction at the interface of a polyisocyanate 1 with the amine groups of both (a) a first polyurethane/polyurea polymer (prepolymer 1), and (b) a water-soluble polyamine 1;
the oil droplets comprise the internal water droplets dispersed therein and a second polyurethane-polyurea polymeric wall;
the second polyurethane-polyurea polymeric wall is obtainable in an oil-in water emulsification process by phase inversion through its reaction at the interface of a polyamine 2 with the isocyanate groups of both (a) a second polyurethane/polyurea polymer (prepolymer 2) and (b) a polyisocyanate 2;
both prepolymers, prepolymer 1 and prepolymer 2, have a main chain and side chains of different polarities;
the main chains of both prepolymers comprises in its backbone urethane functional groups, urea functional groups, polyethylene oxide repeating units, disulfide bonds, amine bonds, and optionally hydrophobic repeating units; and two end terminals functional groups;
the two-end terminal functional groups of prepolymer 1 are amine terminal groups; and
the two-ended terminal functional groups of prepolymer 2 are isocyanate terminal groups
the prepolymer 2 is obtainable *in situ* from a prepolymer 3 which is the corresponding precursor with secondary amine end terminal groups instead of isocyanate groups.

2. The multi-walled water-in-oil-in-water nanoencapsulate according to claim 1, with a hydrodynamic diameter comprised in the range 20-500 nm measured by dynamic light scattering.

3. The multi-walled water-in-oil-in water nanoencapsulate according to any of the claims 1-2, wherein the polyurethane-polyurea polymeric walls are independently functionalized with a group selected from a diol ester group, a diol fluor containing group, a diamine fluor containing group, a polyalcohol carbohydrate group, a polyamine carbohydrate group, a peptide group, a diol ether group, a targeting ligand, a bioligand, and a charged monomer.

4. The multi-walled water-in-oil-in-water nanoencapsulate according to any of the claims 1-3, wherein prepolymer 1 is obtainable by a process comprising reacting: a1) a polyalcohol comprising disulfide bonds; b1) a polyalcohol; c1) a polyamine; and d1) a polyisocyanate, in the following equivalent ratios:
Polyisocyanate d1) / polyalcohol a1 in an equivalent ratio ranging from 1:0.3 to 1:0.6;
Polyisocyanate d1) / polyalcohol b1 in an equivalent ratio ranging from 1:0.05 to 1:0.15; and
Polyisocyanate d1) / polyamine c1 in an equivalent ratio ranging from 1:0.2 to 1:0.8.

5. The multi-walled water-in-oil-in-water nanoencapsulate according to any of the claims 1-4, wherein prepolymer 3 is obtainable by a process which comprises reacting: a2) a polyalcohol comprising disulfide bonds; b2) a polyalcohol; c2) a polyamine; d2) a polyol-polyamine and e2) a polyisocyanate, in the following equivalent ratios:
Polyisocyanate e2) / polyalcohol a2 in an equivalent ratio ranging from 1:0.05 to 1:0.2;
Polyisocyanate e2) / polyalcohol b2 in an equivalent ratio ranging from 1:0.2 to 1:0.6;
Polyisocyanate e2) / polyamine c2 in an equivalent ratio ranging from 1: 0.2 to 1:0.8; and
Polyisocyanate e2) / diol-polyamine d2 in an equivalent ratio ranging from 1:0.05 to 1:0.3.

6. The multi-walled water-in-oil-in-water nanoencapsulate according to any of the claims 1-5, wherein the oil droplet further comprises a chemical/biological active compound solubilized in the oil phase.

7. A process for the preparation of water-in-oil-in-water multi-walled polymeric nanoencapsulate as defined in any of the claims 1-6, which comprises the following steps:
a) first carrying out a first encapsulation water-in-oil by a process comprising the following steps:
a1) Mixing a water-soluble chemical/biological active compound, a prepolymer 1), and a water-soluble polyamine 1, in a given volume of water;
a2) Adding between 5 to 7-fold higher amount of an organic solvent immiscible in water to generate a first emulsion by means of a phase inversion;
a3) Adding a polyisocyanate 1 soluble in the organic solvent selected for the emulsion to create the wall of the first encapsulation for the water-soluble chemical/biological agent through its stoichiometric reaction at the interface with the amines of the prepolymer 1 and the water-soluble polyamine 1;
b) Carrying out a second encapsulation oil-in-water by a process comprising the following steps:
b1) Adding to the previous encapsulation a prepolymer 3 which is the corresponding precursor of prepolymer 2 with secondary amine terminal groups instead of isocyanate groups;
b2) Adding the mixture of step b1) over an equivalent excess of polyisocyanate 2 with respect to the equivalent amount of the prepolymer 3 to form a prepolymer 2 *in situ*;
b3) Adding the necessary amount of water to generate a second emulsion by means of a second phase inversion;
b4) Adding a water-soluble polyamine 2 to create the wall of the second encapsulation through its reaction at the interface with the isocyanate from prepolymer 2 and the free polyisocyanate 2 added in excess;
c) Optionally, evaporating the organic solvents;
d) Optionally, dialyzing the water in oil in water emulsion of step c);
wherein prepolymer 1, prepolymer 2, and prepolymer 3 have independently a main chain and side chains of different polarities;
the main chains of each of the prepolymers comprises in its backbone urethane functional groups, urea functional groups, polyethylene oxide repeating units, disulfide bonds, amine bonds, and optionally hydrophobic repeating units; and two end terminals functional groups;
the two-end terminal functional groups of prepolymer 1 are secondary amine terminal groups; and
the two end terminal functional groups of prepolymer 2 are isocyanate terminal groups; and
the two ended terminal functional groups of prepolymer 3 are secondary amine terminal groups.

8. The process according to claim 7, wherein the solvent used in the oil phase of the first encapsulation comprises a water immiscible solvent.

9. A multi-walled nanoencapsulate obtainable by the process of any of the claims 7-8, in which step c) is performed.

10. A hybrid water-in-oil-in-water polyurethane/polyurea liposome comprising internal water droplets which are dispersed within larger oil droplets.
wherein:
a) the internal water droplets comprise a chemical/biological
active compound solubilized in the water and a first polyurethane-polyurea polymeric wall; the first polyurethane-polyurea polymeric wall is obtainable in a water-in-oil emulsification process by phase inversion through reaction at the interface of a polyisocyanate 1 with the amine groups of both (a) a first polyurethane/polyurea polymer (prepolymer 1), and (b) a water-soluble polyamine 1;
prepolymer 1 have a main chain and side chains of different polarities;
the main chains comprise in its backbone urethane functional groups, urea functional groups, polyethylene oxide repeating units, disulfide bonds, amine bonds and optionally hydrophobic tailored repeating units; and two end terminal functional groups;
the two-end terminal functional groups of prepolymer 1 are secondary amine terminal groups;
b) the oil droplets comprise the internal water droplets dispersed therein and an outer wall which resembles the structure of a liposome;
the outer wall is obtainable in an oil-in-water emulsification through surface arrangement of di-palmitoyl-phosphatidylcholine and cholesterol.

11. A process for the preparation of a hybrid water-in-oil-in-water polyurethane/polyurea liposome comprising internal water droplets which are dispersed within larger oil droplets as defined in claim 10 which comprises the following steps:
a) first carrying out a first encapsulation water-in-oil by a process comprising the following steps: a1) Dissolving a water-soluble chemical/biological active compound, a prepolymer 1), and a water-soluble polyamine 1, in a given volume of water; a2) Adding the 5 to 7-fold more amount of an organic solvent immiscible in water to generate a first emulsion by means of a phase inversion; a3) Adding a polyisocyanate 1 soluble in the organic solvent selected for the emulsion to create the wall of the first encapsulation for the water-soluble chemical/biological agent through its reaction at the interface with the amines of the prepolymer 1 and the water-soluble polyamine 1;
wherein prepolymer 1 have a main chain and side chains of different polarities; the main chains of each of the prepolymer 1 comprises in its backbone urethane functional groups, urea functional groups, polyethylene oxide repeating units, disulfide bonds, and amine bonds, and optionally hydrophobic tailored repeating units; and two end terminal functional groups; the two-end terminal functional groups of prepolymer 1 are secondary amine terminal groups;
b) carrying out a second encapsulation oil-in-water by a process comprising the following steps:
b1) Adding to the previous encapsulation di-palmitoyl-phosphatidylcholine in a first suitable solvent and cholesterol in a second suitable solvent;
b2) Adding the necessary amount of water to generate a second emulsion by means of a second phase inversion and create and outer wall through physical rearrangement of di-palmitoyl-phosphatidylcholine and cholesterol;
c) Optionally, evaporating the organic solvents;
d) Optionally, dialyzing the water in oil in water emulsion of step c);

12. A hybrid polyurethane/polyurea liposome obtainable by the process of claim 11 in which step c) is performed.

13. A composition comprising a nano encapsulate as defined in any of the claims 1-6 or 9, or the hybrid water-in-oil-in-water polyurethane/polyurea liposome according to any of the claims 10 or 12, together with carriers.

14. The composition according to claim 13 which is a cosmetic or pharmaceutical composition comprising a nano encapsulate as defined in any of the claims 1-6 or 9, or the liposome according to any of the claims 10 or 12, together with cosmetically or pharmaceutically acceptable excipients and/or carriers.

## Patentansprüche

1. Ein mehrwandiges Wasser-in-Öl-in-Wasser-Nanoverkapselungsmaterial, umfassend innere Wassertröpfchen, die in Öltröpfchen dispergiert sind, die ihrerseits in einer äußeren wässrigen kontinuierlichen Phase dispergiert sind;
wobei:
die inneren Wassertröpfchen eine im Wasser aufgelöste chemische/biologische aktive Verbindung und eine erste Polyurethan-Polyharnstoff-Polymerwand umfassen;
die erste Polyurethan-Polyharnstoff-Polymerwand in einem Wasser-in-Öl-Emulgierverfahren durch Phasenumkehr durch Reaktion an der Grenzfläche eines Polyisocyanats 1 mit den Amingruppen sowohl (a) eines ersten Polyurethan/Polyharnstoff-Polymers (Prepolymer 1) als auch (b) eines wasserlöslichen Polyamins 1 erhalten werden kann;
die Öltröpfchen die darin dispergierten inneren Wassertröpfchen und eine zweite Polyurethan-Polyharnstoff-Polymerwand umfassen;
die zweite Polyurethan-Polyharnstoff-Polymerwand in einem Öl-in-Wasser-Emulgierverfahren durch Phasenumkehr durch ihre Reaktion an der Grenzfläche eines Polyamins 2 mit den Isocyanatgruppen sowohl (a) eines zweiten Polyurethan/Polyharnstoff-Polymers (Prepolymer 2) als auch (b) eines Polyisocyanats 2 erhalten werden kann;
beide Prepolymere, das Prepolymer 1 und das Prepolymer 2, eine Hauptkette und Seitenketten unterschiedlicher Polarität haben;
die Hauptketten beider Prepolymere in ihrem Rückgrat funktionelle Urethangruppen, funktionelle Harnstoffgruppen, Polyethylenoxid-Wiederholungseinheiten, Disulfidbindungen, Aminbindungen und wahlweise hydrophobe Wiederholungseinheiten; und zweiendige endständige funktionelle Gruppen umfassen;
die zweiendigen endständigen funktionellen Gruppen des Prepolymers 1 endständige Amingruppen sind; und
die zweiendigen endständigen funktionellen Gruppen des Prepolymers 2 endständige Isocyanatgruppen sind
das Prepolymer 2 *in situ* aus einem Prepolymer 3 erhalten werden kann, bei dem es sich um die entsprechende Vorstufe mit Endgruppen, die endständigen sekundären Amingruppen sind, anstelle von Isocyanatgruppen handelt.

2. Das mehrwandige Wasser-in-Öl-in-Wasser-Nanoverkapselungsmaterial nach Anspruch 1, mit einem hydrodynamischen Durchmesser im Bereich von 20-500 nm, gemessen durch dynamische Lichtstreuung.

3. Das mehrwandige Wasser-in-Öl-in-Wasser-Nanoverkapselungsmaterial nach einem der Ansprüche 1 bis 2, wobei die Polyurethan-Polyharnstoff-Polymerwände unabhängig voneinander mit einer Gruppe funktionalisiert sind, die aus einer Diolestergruppe, einer Diolfluor enthaltenden Gruppe, einer Diaminfluor enthaltenden Gruppe, einer Polyalkoholkohlenhydratgruppe, einer Polyaminkohlenhydratgruppe, einer Peptidgruppe, einer Diolethergrup, einem Zielliganden, einem Bioliganden und einem geladenen Monomer ausgewählt ist.

4. Das mehrwandige Wasser-in-Öl-in-Wasser-Nanoverkapselungsmaterial nach einem der Ansprüche 1 bis 3, wobei das Prepolymer 1 durch ein Verfahren erhalten werden kann, welches die Reaktion umfasst von: a1) einem Polyalkohol, der Disulfidbindungen umfasst; b1) einem Polyalkohol; c1) einem Polyamin; und d1) einem Polyisocyanat, in den unten angegebenen Äquivalentverhältnissen:
Polyisocyanat d1) / Polyalkohol a1 in einem Äquivalentverhältnis, das von 1:0,3 bis 1:0,6 reicht;
Polyisocyanat d1) / Polyalkohol b1 in einem Äquivalentverhältnis, das von 1:0,05 bis 1:0,15 reicht; und
Polyisocyanat d1) / Polyamin c1 in einem Äquivalentverhältnis, das von 1:0,2 bis 1:0,8 reicht.

5. Das mehrwandige Wasser-in-Öl-in-Wasser-Nanoverkapselungsmaterial nach einem der Ansprüche 1 bis 4, wobei das Prepolymer 3 durch ein Verfahren erhalten werden kann, welches die Reaktion umfasst von: a2) einem Polyalkohol, der Disulfidbindungen umfasst; b2) einem Polyalkohol; c2) einem Polyamin; d2) einem Polyol-Polyamin und e2) einem Polyisocyanat, in den unten angegebenen Äquivalentverhältnissen:
Polyisocyanat e2) / Polyalkohol a2 in einem Äquivalentverhältnis, das von 1:0,05 bis 1:0,2 reicht;
Polyisocyanat e2) / Polyalkohol b2 in einem Äquivalentverhältnis, das von 1:0,2 bis 1:0,6 reicht;
Polyisocyanat e2) / Polyamin c2 in einem Äquivalentverhältnis, das von 1: 0,2 bis 1:0,8 reicht; und
Polyisocyanat e2) /Diol-Polyamin d2 in einem Äquivalentverhältnis, das von 1:0,05 bis 1:0,3 reicht.

6. Das mehrwandige Wasser-in-Öl-in-Wasser-Nanoverkapselungsmaterial nach einem der Ansprüche 1 bis 5, wobei das Öltröpfchen ferner eine chemische/biologische aktive Verbindung umfasst, die in der Ölphase aufgelöst ist.

7. Ein Verfahren zur Herstellung von Wasser-in-Öl-in-Wasser mehrwandigem polymerem Nanoverkapselungsmaterial wie in einem der Ansprüche 1 bis 6 definiert, das die folgenden Schritte umfasst:
a) zuerst Durchführen einer ersten Verkapselung, Wasser-in-Öl, durch ein Verfahren, das die folgenden Schritte umfasst:
a1) Mischen eines wasserlöslichen chemischen/biologischen Wirkstoffs, eines Prepolymers 1) und eines wasserlöslichen Polyamins 1 in einem bestimmten Wasservolumen;
a2) Zugeben einer zwischen 5- bis 7-fach höheren Menge eines organischen Lösungsmittels, das mit Wasser nicht mischbar ist, um eine erste Emulsion mittels einer Phasenumkehr zu erzeugen;
a3) Zugeben eines in dem für die Emulsion ausgewählten organischen Lösungsmittel löslichen Polyisocyanats 1, um die Wand des ersten Verkapselungsmaterials für den wasserlöslichen chemischen/biologischen Wirkstoff durch seine stöchiometrische Reaktion an der Grenzfläche mit den Aminen des Prepolymers 1 und des wasserlöslichen Polyamins 1 zu erzeugen;
b) Durchführen einer zweiten Verkapselung, Öl-in-Wasser, durch ein Verfahren, das die folgenden Schritte umfasst:
b1) Zugeben eines Prepolymers 3, das die entsprechende Vorstufe des Prepolymers 2 mit sekundären endständigen Amingruppen anstelle von Isocyanatgruppen ist, zu dem vorherigen Verkapselungsmaterial;
b2) Zugeben der Mischung aus Schritt b1) über einen äquivalenten Überschuss an Polyisocyanat 2 in Bezug auf die äquivalente Menge des Prepolymers 3, um ein Prepolymer 2 *in situ* zu bilden;
b3) Zugeben der notwendigen Wassermenge zur Erzeugung einer zweiten Emulsion mittels einer zweiten Phasenumkehr;
b4) Zugeben eines wasserlöslichen Polyamins 2, um die Wand des zweiten Verkapselungsmaterials durch seine Reaktion an der Grenzfläche mit dem Isocyanat von dem Prepolymer 2 und dem im Überschuss zugegebenen freien Polyisocyanat 2 zu erzeugen;
c) Wahlweise, Verdampfen der organischen Lösungsmittel;
d) Wahlweise, Dialyse der Wasser-in-Öl-in-Wasser-Emulsion von Schritt c);
wobei das Prepolymer 1, das Prepolymer 2 und das Prepolymer 3 unabhängig voneinander eine Hauptkette und Seitenketten unterschiedlicher Polarität haben;
die Hauptketten jedes der Prepolymere in seinem Rückgrat funktionelle Urethangruppen, funktionelle Harnstoffgruppen, Polyethylenoxid-Wiederholungseinheiten, Disulfidbindungen, Aminbindungen und wahlweise hydrophobe Wiederholungseinheiten; und zweiendige endständige funktionelle Gruppen umfassen;
die zweiendigen endständigen funktionellen Gruppen des Prepolymers 1 sekundäre endständige Amingruppen sind; und
die zweiendigen endständigen funktionellen Gruppen des Prepolymers 2 endständige Isocyanatgruppen sind; und
die zweiendigen endständigen funktionellen Gruppen des Prepolymers 3 sekundäre endständige Amingruppen sind.

8. Das Verfahren nach Anspruch 7, wobei das in der Ölphase des ersten Verkapselungsmaterials verwendete Lösungsmittel ein mit Wasser nicht mischbares Lösungsmittel umfasst.

9. Ein mehrwandiges Nanoverkapselungsmaterial, das durch das Verfahren von einem der Ansprüche 7 bis 8 erhalten werden kann, bei dem Schritt c) durchgeführt wird.

10. Ein hybrides Wasser-in-ÖI-in-Wasser-Polyurethan/Polyharnstoff-Liposom umfassend interne Wassertröpfchen, die in größeren Öltröpfchen dispergiert sind.
wobei:
a) die inneren Wassertröpfchen eine chemische/biologische aktive Verbindung, die im Wasser aufgelöst ist, und eine erste Polyurethan-Polyharnstoff-Polymerwand umfassen;
die erste Polyurethan-Polyharnstoff-Polymerwand in einem Wasser-in-Öl-Emulgierverfahren durch Phasenumkehr durch Reaktion an der Grenzfläche eines Polyisocyanats 1 mit den Amingruppen sowohl (a) eines ersten Polyurethan/Polyharnstoff-Polymers (Prepolymer 1) als auch (b) eines wasserlöslichen Polyamins 1 erhalten werden kann;
die Prepolymer 1 eine Hauptkette und Seitenketten unterschiedlicher Polaritäten haben;
die Hauptketten in ihrem Rückgrat funktionelle Urethangruppen, funktionelle Harnstoffgruppen, Polyethylenoxid-Wiederholungseinheiten, Disulfidbindungen, Aminbindungen und wahlweise hydrophobe spezifisch angepasste Wiederholungseinheiten; und zweiendige endständige funktionelle Gruppen umfassen;
die zweiendigen endständigen funktionellen Gruppen des Prepolymers 1 sekundäre endständige Amingruppen sind;
b) die Öltröpfchen die darin dispergierten inneren Wassertröpfchen und eine Außenwand umfassen, die der Struktur eines Liposoms ähnelt;
die Außenwand in einer Öl-in-Wasser-Emulgierung durch Oberflächenanordnung von Dipalmitoylphosphatidylcholin und Cholesterin erhalten werden kann.

11. Ein Verfahren zur Herstellung eines hybriden Wasser-in-Öl-in-Wasser-Polyurethan/Polyharnstoff-Liposoms, das interne Wassertröpfchen umfasst, die in größeren Öltröpfchen wie in Anspruch 10 definiert dispergiert sind, welches die folgenden Schritte umfasst:
a) zunächst Durchführen einer ersten Verkapselung, Wasser-in-Öl, durch ein Verfahren umfassend die folgenden Schritte: a1) Lösen einer wasserlöslichen chemischen/biologischen aktiven Verbindung, eines Prepolymers 1) und eines wasserlöslichen Polyamins 1, in einem gegebenen Wasservolumen; a2) Zugeben der 5-bis 7-fach höheren Menge eines in Wasser nicht mischbaren organischen Lösungsmittels zur Erzeugung einer ersten Emulsion mittels einer Phasenumkehr; a3) Zugeben eines in dem für die Emulsion ausgewählten organischen Lösungsmittel löslichen Polyisocyanats 1 zur Erzeugung der Wand des ersten Verkapselungsmaterials für den wasserlöslichen chemischen/biologischen Wirkstoff durch dessen Reaktion an der Grenzfläche mit den Aminen des Prepolymers 1 und des wasserlöslichen Polyamins 1;
wobei das Prepolymer 1 eine Hauptkette und Seitenketten mit unterschiedlichen Polaritäten hat; die Hauptketten jedes des Prepolymers 1 in seinem Rückgrat funktionelle Urethangruppen, funktionelle Harnstoffgruppen, Polyethylenoxid-Wiederholungseinheiten, Disulfidbindungen und Aminbindungen und wahlweise hydrophobe spezifisch angepasste Wiederholungseinheiten; und zweiendige endständige funktionelle Gruppen umfasst; wobei die zweiendigen endständigen funktionellen Gruppen des Prepolymers 1 endständige sekundäre Amingruppen sind;
b) Durchführen einer zweiten Verkapselung, Öl-in-Wasser, durch ein Verfahren, das die folgenden Schritte umfasst:
b1) Zugeben von Dipalmitoylphosphatidylcholin in einem ersten geeigneten Lösungsmittel und Cholesterin in einem zweiten geeigneten Lösungsmittel zu dem vorherigen Verkapselungsmaterial;
b2) Zugeben der erforderlichen Menge Wasser, um eine zweite Emulsion durch eine zweite Phasenumkehr zu erzeugen und eine Außenwand durch physikalische Umlagerung von Dipalmitoylphosphatidylcholin und Cholesterin zu schaffen;
c) Wahlweise, Verdampfen der organischen Lösungsmittel;
d) Wahlweise, Dialyse der Wasser-in-Öl-in-Wasser-Emulsion von Schritt c);

12. Ein hybrides Polyurethan/Polyharnstoff-Liposom, das durch das Verfahren von Anspruch 11 erhalten werden kann, bei dem Schritt c) durchgeführt wird.

13. Eine Zusammensetzung umfassend ein Nanoverkapselungsmittel wie in einem der Ansprüche 1 bis 6 oder 9 definiert oder das hybride Wasser-in-Öl-in-Wasser-Polyurethan/Polyharnstoff-Liposom nach einem der Ansprüche 10 oder 12, zusammen mit Trägersubstanzen.

14. Die Zusammensetzung nach Anspruch 13, die eine kosmetische oder pharmazeutische Zusammensetzung ist, die ein Nanoverkapselungsmittel, wie in einem der Ansprüche 1 bis 6 oder 9 definiert, oder das Liposom nach einem der Ansprüche 10 oder 12, zusammen mit kosmetisch oder pharmazeutisch akzeptablen Hilfsstoffen und/oder Trägersubstanzen umfasst.

## Revendications

1. Un nanoencapsulé eau dans l'huile dans l'eau à parois multiples comprenant des gouttelettes d'eau internes qui sont dispersées à l'intérieur de gouttelettes d'huile, elles-mêmes dispersées dans une phase continue aqueuse externe ;
dans lequel :
les gouttelettes d'eau internes comprennent un composé actif chimique/biologique solubilisé dans l'eau et une première paroi polymère de polyuréthane-polyurée ;
la première paroi polymère de polyuréthane-polyurée peut être obtenue dans un procédé d'émulsification eau dans huile par inversion de phases par réaction à l'interface d'un polyisocyanate 1 avec les groupes amine de tant (a) un premier polymère de polyuréthane/polyurée (prépolymère 1) que (b) une polyamine 1 soluble dans l'eau ;
les gouttelettes d'huile comprennent les gouttelettes d'eau internes dispersées dans celles-ci et une seconde paroi polymère de polyuréthane-polyurée ;
la seconde paroi polymère de polyuréthane-polyurée peut être obtenue dans un procédé d'émulsification huile dans l'eau par inversion de phases par sa réaction à l'interface d'une polyamine 2 avec les groupes isocyanate à la fois (a) d'un second polymère polyuréthane/polyurée (prépolymère 2) et (b) d'un polyisocyanate 2 ;
les deux prépolymères, le prépolymère 1 et le prépolymère 2, ont une chaîne principale et des chaînes latérales de polarités différentes ;
les chaînes principales des deux prépolymères comprennent dans son squelette des groupes fonctionnels uréthane, des groupes fonctionnels urée, des motifs de répétition d'oxyde de polyéthylène, des liaisons disulfure, des liaisons amine et facultativement des motifs de répétition hydrophobes ; et des groupes fonctionnels terminaux à deux extrémités ;
les groupes fonctionnels terminaux à deux extrémités de prépolymère 1 sont des groupes terminaux amine ; et
les groupes fonctionnels terminaux à deux extrémités de prépolymère 2 sont des groupes terminaux isocyanate
le prépolymère 2 peut être obtenu *in situ* à partir d'un prépolymère 3 qui est le précurseur correspondant avec des groupes terminaux à extrémité amine secondaire au lieu de groupes isocyanate.

2. Le nanoencapsulé eau dans l'huile dans l'eau à parois multiples selon la revendication 1, avec un diamètre hydrodynamique compris dans la plage de 20 à 500 nm mesuré par diffusion dynamique de la lumière.

3. Le nanoencapsulé eau dans l'huile dans l'eau à parois multiples selon l'une quelconque des revendications 1 à 2, dans lequel les parois polymères de polyuréthane-polyurée sont fonctionnalisées indépendamment avec un groupe choisi parmi un groupe ester de diol, un groupe contenant du diol fluoré, un groupe contenant de la diamine fluorée, un groupe hydrate de carbone de polyalcool, un groupe hydrate de carbone de polyamine, un groupe peptide, un groupe éther de diol, un ligand de ciblage, un bioligand et un monomère chargé.

4. Le nanoencapsulé eau dans l'huile dans l'eau à parois multiples selon l'une quelconque des revendications 1 à 3, dans lequel le prépolymère 1 peut être obtenu par un procédé comprenant la réaction : a1) d'un polyalcool comprenant des liaisons disulfure ; b1) d'un polyalcool ; c1) d'une polyamine ; et d1) d'un polyisocyanate, dans les rapports d'équivalents suivants :
Polyisocyanate d1) / polyalcool a1 dans un rapport d'équivalents allant de 1:0,3 à 1:0,6 ;
Polyisocyanate d1) / polyalcool b1 dans un rapport d'équivalents allant de 1:0,05 à 1:0,15 ; et
Polyisocyanate d1) / polyamine c1 dans un rapport d'équivalents allant de 1:0,2 à 1:0,8.

5. Le nanoencapsulé eau dans l'huile dans l'eau à parois multiples selon l'une quelconque des revendications 1 à 4, dans lequel le prépolymère 3 peut être obtenu par un procédé qui comprend la réaction : a2) d'un polyalcool comprenant des liaisons disulfure ; b2) d'un polyalcool ; c2) d'une polyamine ; d2) d'un polyol-polyamine et e2) d'un polyisocyanate, dans les rapports d'équivalents suivants :
Polyisocyanate e2) / polyalcool a2 dans un rapport d'équivalents allant de 1:0,05 à 1:0,2 ;
Polyisocyanate e2) / polyalcool b2 dans un rapport d'équivalents allant de 1:0,2 à 1:0,6 ;
Polyisocyanate e2) / polyamine c2 dans un rapport d'équivalents allant de 1 : 0,2 à 1:0,8 ; et
Polyisocyanate e2) / diol-polyamine d2 dans un rapport d'équivalents allant de 1:0,05 à 1:0,3.

6. Le nanoencapsulé eau dans l'huile dans l'eau à parois multiples selon l'une quelconque des revendications 1 à 5, dans lequel la gouttelette d'huile comprend en outre un composé actif chimique/biologique solubilisé dans la phase huileuse.

7. Un procédé de préparation d'un nanoencapsulé polymère à parois multiples d'eau dans l'huile dans l'eau tel que défini dans l'une quelconque des revendications 1 à 6, qui comprend les étapes suivantes :
a) d'abord réaliser une première encapsulation eau dans l'huile par un procédé comprenant les étapes suivantes :
a1) Mélanger un composé actif chimique/biologique soluble dans l'eau, un prépolymère 1) et une polyamine 1 soluble dans l'eau, dans un volume donné d'eau ;
a2) Ajouter entre 5 à 7 fois plus d'un solvant organique non miscible dans l'eau pour générer une première émulsion au moyen d'une inversion de phases ;
a3) Ajouter un polyisocyanate 1 soluble dans le solvant organique sélectionné pour l'émulsion afin de créer la paroi de la première encapsulation pour l'agent chimique/biologique soluble dans l'eau par sa réaction stoechiométrique à l'interface avec les amines du prépolymère 1 et de la polyamine 1 soluble dans l'eau ;
b) Réaliser une seconde encapsulation huile dans l'eau par un procédé comprenant les étapes suivantes :
b1) Ajouter à l'encapsulation précédente un prépolymère 3 qui est le précurseur correspondant du prépolymère 2 avec des groupes terminaux amine secondaire au lieu de groupes isocyanate ;
b2) Ajouter le mélange de l'étape b1) sur un excès équivalent de polyisocyanate 2 par rapport à la quantité équivalente du prépolymère 3 pour former un prépolymère 2 *in situ* ;
b3) Ajouter la quantité d'eau nécessaire pour générer une seconde émulsion au moyen d'une seconde inversion de phases ;
b4) Ajouter une polyamine 2 soluble dans l'eau pour créer la paroi de la seconde encapsulation par sa réaction à l'interface avec l'isocyanate du prépolymère 2 et le polyisocyanate 2 libre ajouté en excès ;
c) Facultativement, évaporer les solvants organiques ;
d) Facultativement, dialyser l'émulsion eau dans huile dans l'eau de l'étape c) ;
dans lequel le prépolymère 1, le prépolymère 2 et le prépolymère 3 ont indépendamment une chaîne principale et des chaînes latérales de polarités différentes ;
les chaînes principales de chacun des prépolymères comprennent dans son squelette des groupes fonctionnels uréthane, des groupes fonctionnels urée, des motifs de répétition d'oxyde de polyéthylène, des liaisons disulfure, des liaisons amine et facultativement des motifs de répétition hydrophobes ; et des groupes fonctionnels terminaux à deux extrémités ;
les groupes fonctionnels terminaux à deux extrémités de prépolymère 1 sont des groupes terminaux amine secondaire ; et
les groupes fonctionnels terminaux à deux extrémités de prépolymère 2 sont des groupes terminaux isocyanate ; et
les groupes fonctionnels terminaux à deux extrémités de prépolymère 3 sont des groupes terminaux amine secondaire.

8. Le procédé selon la revendication 7, dans lequel le solvant utilisé dans la phase huileuse de la première encapsulation comprend un solvant non miscible dans l'eau.

9. Un nanoencapsulé à parois multiples pouvant être obtenu par le procédé de l'une quelconque des revendications 7 à 8, dans lequel l'étape c) est réalisée.

10. Un liposome hybride polyuréthane/polyurée eau dans l'huile dans l'eau comprenant des gouttelettes d'eau internes qui sont dispersées dans des gouttelettes d'huile plus grosses.
dans lequel :
a) les gouttelettes d'eau internes comprennent un composé actif chimique/biologique solubilisé dans l'eau et une première paroi polymère de polyuréthane-polyurée ;
la première paroi polymère de polyuréthane-polyurée peut être obtenue dans un procédé d'émulsification eau dans l'huile par inversion de phases par réaction à l'interface d'un polyisocyanate 1 avec les groupes amine de tant (a) un premier polymère de polyuréthane/polyurée (prépolymère 1) que de (b) une polyamine 1 soluble dans l'eau ;
les prépolymère 1 ont une chaîne principale et des chaînes latérales de polarités différentes ;
les chaînes principales comprennent dans son squelette des groupes fonctionnels uréthane, des groupes fonctionnels urée, des motifs de répétition d'oxyde de polyéthylène, des liaisons disulfure, des liaisons amine et facultativement des motifs de répétition spécifiquement adaptés hydrophobes ; et des groupes fonctionnels terminaux à deux extrémités ;
les groupes fonctionnels terminaux à deux extrémités de prépolymère 1 sont des groupes terminaux amine secondaire ;
b) les gouttelettes d'huile comprennent les gouttelettes d'eau internes dispersées dans celles-ci et une paroi externe qui ressemble à la structure d'un liposome ;
la paroi extérieure peut être obtenue dans une émulsification huile dans l'eau par arrangement de surface de dipalmitoyl-phosphatidylcholine et cholestérol.

11. Un procédé de préparation d'un liposome hybride polyuréthane/polyurée eau dans l'huile dans l'eau comprenant des gouttelettes d'eau internes qui sont dispersées dans des gouttelettes d'huile plus grosses tel que défini dans la revendication 10, qui comprend les étapes suivantes :
a) réaliser d'abord une première encapsulation eau dans l'huile par un procédé comprenant les étapes suivantes : a1) Dissoudre un composé actif chimique/biologique soluble dans l'eau, un prépolymère 1) et une polyamine 1 soluble dans l'eau, dans un volume donné d'eau ; a2) Ajouter une quantité de 5 à 7 fois plus importante d'un solvant organique non miscible dans l'eau pour générer une première émulsion au moyen d'une inversion de phases ; a3) Ajouter un polyisocyanate 1 soluble dans le solvant organique sélectionné pour l'émulsion pour créer la paroi de la première encapsulation pour l'agent chimique/biologique soluble dans l'eau par sa réaction à l'interface avec les amines du prépolymère 1 et de la polyamine 1 soluble dans l'eau ;
dans lequel les prépolymère 1 ont une chaîne principale et des chaînes latérales de polarités différentes ; les chaînes principales de chacun des prépolymères 1 comprennent dans son squelette des groupes fonctionnels uréthane, des groupes fonctionnels urée, des motifs de répétition d'oxyde de polyéthylène, des liaisons disulfure et des liaisons amine, et facultativement des motifs de répétition adaptées hydrophobes ; et des groupes fonctionnels terminaux à deux extrémités ; les groupes fonctionnels terminaux à deux extrémités de prépolymère 1 sont des groupes terminaux amine secondaire ;
b) réaliser une seconde encapsulation huile dans l'eau par un procédé comprenant les étapes suivantes :
b1) Ajouter à l'encapsulation précédente de la dipalmitoyl-phosphatidylcholine dans un premier solvant approprié et du cholestérol dans un second solvant approprié ;
b2) Ajouter la quantité d'eau nécessaire pour générer une seconde émulsion au moyen d'une seconde inversion de phases et créer une paroi externe par réarrangement physique de la dipalmitoyl-phosphatidylcholine et du cholestérol ;
c) Facultativement, évaporer les solvants organiques ;
d) Facultativement, dialyser l'émulsion eau dans huile dans l'eau de l'étape c) ;

12. Un liposome hybride polyuréthane/polyurée pouvant être obtenu par le procédé de la revendication 11 dans lequel l'étape c) est réalisée.

13. Une composition comprenant un nanoencapsulé tel que défini dans l'une quelconque des revendications 1 à 6 ou 9, ou le liposome hybride polyuréthane/polyurée eau dans l'huile dans l'eau selon l'une quelconque des revendications 10 ou 12, avec des véhicules.

14. La composition selon la revendication 13, qui est une composition cosmétique ou pharmaceutique comprenant un nanoencapsulé tel que défini dans l'une quelconque des revendications 1 à 6 ou 9, ou le liposome selon l'une quelconque des revendications 10 ou 12, avec des excipients et/ou des véhicules cosmétiquement ou pharmaceutiquement acceptables.
